# EUROPEAN PATENT APPLICATION

(11) **EP 2 508 619 A1**
(43) Date of publication of application: **10.10.2012**
(21) Application number: 11161244.6
(22) Date of filing: 05.04.2011
(51) Int. Cl.: C12Q 1/68

(54) **Method and kits for the prediction of response/nonresponse to the treatment with an anti-EGFR antibody in patients with colorectal cancer of all UICC stages**

(71) Applicant: Signature Diagnostics AG, 14473 Potsdam (DE)
(72) Inventor: Pechanska, Paulina, 14193 Berlin (DE); Hinzmann, Bernd, 13127 Berlin (DE); May, Tobias, 10435 Berlin (DE); Rosenthal, André, 14974 Ludwigsfelde (DE)
(74) Representative: Hoppe, Georg Johannes

(57) **Abstract**

The invention pertains to a method for predicting responsiveness of a human patient in need of treatment with an anti-EGFR antibody to such treatment based on a biological sample. According to the invention, this method comprises: determining the mutation status of KRAS, BRAF and PIK3CA, and concluding based on the mutation status whether the subject will be responsive to treatment with an anti-EGFR antibody.

SEQUENCE LISTING

<110>  Signature Diagnostics AG

<120>  Methods and Kits for the Prediction of Response/Nonresponse to
       the Treatment with an anti-EGFR Antibody in Patients with
       Colorectal Cancer of all UICC Stages

<130>  SIG002EP-2

<160>  5

<170>  PatentIn version 3.5

<210>  1
<211>  3567
<212>  DNA
<213>  Homo sapiens

<400>  1
atgcctccac gaccatcatc aggtgaactg tggggcatcc acttgatgcc cccaagaatc      60

ctagtagaat gtttactacc aaatggaatg atagtgactt tagaatgcct ccgtgaggct     120

acattaataa ccataaagca tgaactattt aaagaagcaa gaaaataccc cctccatcaa     180

cttcttcaag atgaatcttc ttacattttc gtaagtgtta ctcaagaagc agaaagggaa     240

gaattttttg atgaaacaag acgactttgt gaccttcggc tttttcaacc ctttttaaaa     300

gtaattgaac cagtaggcaa ccgtgaagaa aagatcctca atcgagaaat tggttttgct     360

atcggcatgc cagtgtgtga atttgatatg gttaaagatc cagaagtaca ggacttccga     420

agaaatattc tgaacgtttg taaagaagct gtggatctta gggacctcaa ttcacctcat     480

agtagagcaa tgtatgtcta tcctccaaat gtagaatctt caccagaatt gccaaagcac     540

atatataata aattagataa agggcaaata atagtggtga tctgggtaat agtttctcca     600

aataatgaca agcagaagta tactctgaaa atcaaccatg actgtgtacc agaacaagta     660

attgctgaag caatcaggaa aaaaactcga agtatgttgc tatcctctga acaactaaaa     720

ctctgtgttt tagaatatca gggcaagtat attttaaaag tgtgtggatg tgatgaatac     780

ttcctagaaa aatatcctct gagtcagtat aagtatataa gaagctgtat aatgcttggg     840

aggatgccca atttgatgtt gatggctaaa gaaagccttt attctcaact gccaatggac     900

tgttttacaa tgccatctta ttccagacgc atttccacag ctacaccata tatgaatgga     960

gaaacatcta caaaatccct ttgggttata aatagtgcac tcagaataaa aattctttgt    1020

gcaacctacg tgaatgtaaa tattcgagac attgataaga tctatgttcg aacaggtatc    1080

taccatggag gagaaccctt atgtgacaat gtgaacactc aaagagtacc ttgttccaat    1140

cccaggtgga atgaatggct gaattatgat atatacattc ctgatcttcc tcgtgctgct    1200

cgactttgcc tttccatttg ctctgttaaa ggccgaaagg gtgctaaaga ggaacactgt 1260

ccattggcat ggggaaatat aaacttgttt gattacacag acactctagt atctggaaaa 1320

atggctttga atctttggcc agtacctcat ggattagaag atttgctgaa ccctattggt 1380

gttactggat caaatccaaa taaagaaact ccatgcttag agttggagtt tgactggttc 1440

agcagtgtgg taaagttccc agatatgtca gtgattgaag agcatgccaa ttggtctgta 1500

tcccgagaag caggatttag ctattcccac gcaggactga gtaacagact agctagagac 1560

aatgaattaa gggaaaatga caaagaacag ctcaaagcaa tttctacacg agatcctctc 1620

tctgaaatca ctgagcagga gaaagatttt ctatggagtc acagacacta ttgtgtaact 1680

atccccgaaa ttctacccaa attgcttctg tctgttaaat ggaattctag agatgaagta 1740

gcccagatgt attgcttggt aaaagattgg cctccaatca aacctgaaca ggctatggaa 1800

cttctggact gtaattaccc agatcctatg gttcgaggtt ttgctgttcg gtgcttggaa 1860

aaatatttaa cagatgacaa actttctcag tatttaattc agctagtaca ggtcctaaaa 1920

tatgaacaat atttggataa cttgcttgtg agatttttac tgaagaaagc attgactaat 1980

caaaggattg ggcacttttt cttttggcat ttaaaatctg agatgcacaa taaaacagtt 2040

agccagaggt ttggcctgct tttggagtcc tattgtcgtg catgtgggat gtatttgaag 2100

cacctgaata ggcaagtcga ggcaatggaa aagctcatta acttaactga cattctcaaa 2160

caggagaaga aggatgaaac acaaaaggta cagatgaagt ttttagttga gcaaatgagg 2220

cgaccagatt tcatggatgc tctacagggc tttctgtctc ctctaaaccc tgctcatcaa 2280

ctaggaaacc tcaggcttga agagtgtcga attatgtcct ctgcaaaaag gccactgtgg 2340

ttgaattggg agaacccaga catcatgtca gagttactgt ttcagaacaa tgagatcatc 2400

tttaaaaatg gggatgattt acggcaagat atgctaacac ttcaaattat tcgtattatg 2460

gaaaatatct ggcaaaatca aggtcttgat cttcgaatgt taccttatgg ttgtctgtca 2520

atcggtgact gtgtgggact tattgaggtg gtgcgaaatt ctcacactat tatgcaaatt 2580

cagtgcaaag gcggcttgaa aggtgcactg cagttcaaca gccacacact acatcagtgg 2640

ctcaaagaca agaacaaagg agaaatatat gatgcagcca ttgacctgtt tacacgttca 2700

tgtgctggat actgtgtagc taccttcatt ttgggaattg gagatcgtca caatagtaac 2760

atcatggtga aagacgatgg acaactgttt catatagatt ttggacactt tttggatcac 2820

aagaagaaaa aatttggtta taaacgagaa cgtgtgccat ttgttttgac acaggatttc 2880

ttaatagtga ttagtaaagg agcccaagaa tgcacaaaga caagagaatt tgagaggttt 2940

caggagatgt gttacaaggc ttatctagct attcgacagc atgccaatct cttcataaat 3000

cttttctcaa tgatgcttgg ctctggaatg ccagaactac aatcttttga tgacattgca 3060

tacattcgaa agaccctagc cttagataaa actgagcaag aggctttgga gtatttcatg 3120

aaacaaatga atgatgcaca tcatggtggc tggacaacaa aaatggattg gatcttccac 3180

acaattaaac agcatgcatt gaactgaaaa gataactgag aaaatgaaag ctcactctgg 3240

attccacact gcactgttaa taactctcag caggcaaaga ccgattgcat aggaattgca 3300

caatccatga acagcattag aatttacagc aagaacagaa ataaaatact atataattta 3360

aataatgtaa acgcaaacag ggtttgatag cacttaaact agttcatttc aaaattaagc 3420

tttagaataa tgcgcaattt catgttatgc cttaagtcca aaaaggtaaa ctttgaagat 3480

tgtttgtatc tttttttaaa aaacaaaaca aaacaaaaat ccccaaaata tatagaaatg 3540

atggagaagg aaaaaaaaaa aaaaaaa 3567

<210> 2
<211> 5131
<212> DNA
<213> Homo sapiens

<400> 2
atgactgaat ataaacttgt ggtagttgga gctggtggcg taggcaagag tgccttgacg 60

atacagctaa ttcagaatca ttttgtggac gaatatgatc caacaataga ggattcctac 120

aggaagcaag tagtaattga tggagaaacc tgtctcttgg atattctcga cacagcaggt 180

caagaggagt acagtgcaat gagggaccag tacatgagga ctggggaggg ctttctttgt 240

gtatttgcca taaataatac taaatcattt gaagatattc accattatag agaacaaatt 300

aaaagagtta aggactctga agatgtacct atggtcctag taggaaataa atgtgatttg 360

ccttctagaa cagtagacac aaaacaggct caggacttag caagaagtta tggaattcct 420

tttattgaaa catcagcaaa gacaagacag ggtgttgatg atgccttcta tacattagtt 480

cgagaaattc gaaaacataa agaaaagatg agcaaagatg gtaaaaagaa gaaaaagaag 540

tcaaagacaa agtgtgtaat tatgtaaata caatttgtac ttttttctta aggcatacta 600

gtacaagtgg taatttttgt acattacact aaattattag catttgtttt agcattacct 660

aatttttttc ctgctccatg cagactgtta gcttttacct taaatgctta ttttaaaatg 720

acagtggaag tttttttttc ctctaagtgc cagtattccc agagttttgg tttttgaact 780

agcaatgcct gtgaaaaaga aactgaatac ctaagatttc tgtcttgggg tttttggtgc 840

atgcagttga ttacttctta tttttcttac caattgtgaa tgttggtgtg aaacaaatta 900

atgaagcttt tgaatcatcc ctattctgtg ttttatctag tcacataaat ggattaatta 960

ctaatttcag ttgagacctt ctaattggtt tttactgaaa cattgaggga acacaaattt 1020

atgggcttcc tgatgatgat tcttctaggc atcatgtcct atagtttgtc atccctgatg 1080

aatgtaaagt tacactgttc acaaaggttt tgtctccttt ccactgctat tagtcatggt 1140

cactctcccc aaaatattat attttttcta taaaaagaaa aaaatggaaa aaaattacaa 1200

ggcaatggaa actattataa ggccatttcc ttttcacatt agataaatta ctataaagac 1260

tcctaatagc ttttcctgtt aaggcagacc cagtatgaaa tggggattat tatagcaacc 1320

attttggggc tatatttaca tgctactaaa tttttataat aattgaaaag attttaacaa 1380

gtataaaaaa ttctcatagg aattaaatgt agtctccctg tgtcagactg ctctttcata 1440

gtataacttt aaatcttttc ttcaacttga gtctttgaag atagttttaa ttctgcttgt 1500

gacattaaaa gattatttgg gccagttata gcttattagg tgttgaagag accaaggttg 1560

caaggccagg ccctgtgtga acctttgagc tttcatagag agtttcacag catggactgt 1620

gtccccacgg tcatccagtg ttgtcatgca ttggttagtc aaaatgggga gggactaggg 1680

cagtttggat agctcaacaa gatacaatct cactctgtgg tggtcctgct gacaaatcaa 1740

gagcattgct tttgtttctt aagaaaacaa actctttttt aaaaattact tttaaatatt 1800

aactcaaaag ttgagatttt ggggtggtgg tgtgccaaga cattaatttt ttttttaaac 1860

aatgaagtga aaaagtttta caatctctag gtttggctag ttctcttaac actggttaaa 1920

ttaacattgc ataaacactt ttcaagtctg atccatattt aataatgctt taaaataaaa 1980

ataaaaacaa tccttttgat aaatttaaaa tgttacttat tttaaaataa atgaagtgag 2040

atggcatggt gaggtgaaag tatcactgga ctaggaagaa ggtgacttag gttctagata 2100

ggtgtctttt aggactctga ttttgaggac atcacttact atccatttct tcatgttaaa 2160

agaagtcatc tcaaactctt agtttttttt ttttacaact atgtaattta tattccattt 2220

acataaggat acacttattt gtcaagctca gcacaatctg taaattttta acctatgtta 2280

caccatcttc agtgccagtc ttgggcaaaa ttgtgcaaga ggtgaagttt atatttgaat 2340

atccattctc gttttaggac tcttcttcca tattagtgtc atcttgcctc cctaccttcc 2400

acatgcccca tgacttgatg cagttttaat acttgtaatt cccctaacca taagatttac 2460

tgctgctgtg gatatctcca tgaagttttc ccactgagtc acatcagaaa tgccctacat 2520

cttatttcct cagggctcaa gagaatctga cagataccat aaaggatttt gacctaatca 2580

ctaattttca ggtggtggct gatgctttga acatctcttt gctgcccaat ccattagcga 2640

cagtaggatt tttcaaacct ggtatgaata gacagaaccc tatccagtgg aaggagaatt 2700

taataaagat agtgctgaaa gaattcctta ggtaatctat aactaggact actcctggta 2760

acagtaatac attccattgt tttagtaacc agaaatcttc atgcaatgaa aaatacttta 2820

attcatgaag cttacttttt ttttttggtg tcagagtctc gctcttgtca cccaggctgg 2880

aatgcagtgg cgccatctca gctcactgca acctccatct cccaggttca agcgattctc 2940

gtgcctcggc ctcctgagta gctgggatta caggcgtgtg ccactacact caactaattt 3000

ttgtattttt aggagagacg gggtttcacc ctgttggcca ggctggtctc gaactcctga 3060

cctcaagtga ttcacccacc ttggcctcat aaacctgttt tgcagaactc atttattcag 3120

caaatattta ttgagtgcct accagatgcc agtcaccgca caaggcactg ggtatatggt 3180

atccccaaac aagagacata atcccggtcc ttaggtagtg ctagtgtggt ctgtaatatc 3240

ttactaaggc ctttggtata cgacccagag ataacacgat gcgtatttta gttttgcaaa 3300

gaaggggttt ggtctctgtg ccagctctat aattgttttg ctacgattcc actgaaactc 3360

ttcgatcaag ctactttatg taaatcactt cattgtttta aaggaataaa cttgattata 3420

ttgttttttt atttggcata actgtgattc ttttaggaca attactgtac acattaaggt 3480

gtatgtcaga tattcatatt gacccaaatg tgtaatattc cagttttctc tgcataagta 3540

attaaaatat acttaaaaat taatagtttt atctgggtac aaataaacag gtgcctgaac 3600

tagttcacag acaaggaaac ttctatgtaa aaatcactat gatttctgaa ttgctatgtg 3660

aaactacaga tctttggaac actgtttagg tagggtgtta agacttacac agtacctcgt 3720

ttctacacag agaaagaaat ggccatactt caggaactgc agtgcttatg aggggatatt 3780

taggcctctt gaatttttga tgtagatggg cattttttta aggtagtggt taattacctt 3840

tatgtgaact ttgaatggtt taacaaaaga tttgtttttg tagagatttt aaagggggag 3900

aattctagaa ataaatgtta cctaattatt acagccttaa agacaaaaat ccttgttgaa 3960

gtttttttaa aaaaagctaa attacataga cttaggcatt aacatgtttg tggaagaata 4020

tagcagacgt atattgtatc atttgagtga atgttcccaa gtaggcattc taggctctat 4080

ttaactgagt cacactgcat aggaatttag aacctaactt ttataggtta tcaaaactgt 4140

tgtcaccatt gcacaatttt gtcctaatat atacatagaa actttgtggg gcatgttaag 4200

ttacagtttg cacaagttca tctcatttgt attccattga tttttttttt cttctaaaca 4260

ttttttcttc aaacagtata taactttttt taggggattt ttttttagac agcaaaaact 4320

atctgaagat ttccatttgt caaaaagtaa tgatttcttg ataattgtgt agtaatgttt 4380

tttagaaccc agcagttacc ttaaagctga atttatattt agtaacttct gtgttaatac 4440

tggatagcat gaattctgca ttgagaaact gaatagctgt cataaaatga aactttcttt 4500

ctaaagaaag atactcacat gagttcttga agaatagtca taactagatt aagatctgtg 4560

ttttagttta atagtttgaa gtgcctgttt gggataatga taggtaattt agatgaattt 4620

aggggaaaaa aaagttatct gcagatatgt tgagggccca tctctccccc cacaccccca 4680

cagagctaac tgggttacag tgttttatcc gaaagtttcc aattccactg tcttgtgttt 4740

tcatgttgaa aatacttttg catttttcct ttgagtgcca atttcttact agtactattt 4800

cttaatgtaa catgtttacc tggaatgtat tttaactatt tttgtatagt gtaaactgaa 4860

acatgcacat tttgtacatt gtgctttctt ttgtgggaca tatgcagtgt gatccagttg 4920

ttttccatca tttggttgcg ctgacctagg aatgttggtc atatcaaaca ttaaaaatga 4980

ccactctttt aattgaaatt aacttttaaa tgtttatagg agtatgtgct gtgaagtgat 5040

ctaaaatttg taatattttt gtcatgaact gtactactcc taattattgt aatgtaataa 5100

aaatagttac agtgacaaaa aaaaaaaaaa a 5131

<210> 3
<211> 2888
<212> DNA
<213> Homo sapiens

<400> 3
atggcggcgc tgagcggtgg cggtggtggc ggcgcggagc cgggccaggc tctgttcaac 60

ggggacatgg agcccgaggc cggcgccggc gccggcgccg cggcctcttc ggctgcggac 120

cctgccattc cggaggaggt gtggaatatc aaacaaatga ttaagttgac acaggaacat 180

atagaggccc tattggacaa atttggtggg gagcataatc caccatcaat atatctggag 240

gcctatgaag aatacaccag caagctagat gcactccaac aaagagaaca acagttattg 300

gaatctctgg ggaacggaac tgatttttct gtttctagct ctgcatcaat ggataccgtt 360

acatcttctt cctcttctag cctttcagtg ctaccttcat ctctttcagt ttttcaaaat 420

cccacagatg tggcacggag caaccccaag tcaccacaaa aacctatcgt tagagtcttc 480

ctgcccaaca aacagaggac agtggtacct gcaaggtgtg gagttacagt ccgagacagt 540

ctaaagaaag cactgatgat gagaggtcta atcccagagt gctgtgctgt ttacagaatt 600

caggatggag agaagaaacc aattggttgg gacactgata tttcctggct tactggagaa 660

gaattgcatg tggaagtgtt ggagaatgtt ccacttacaa cacacaactt tgtacgaaaa 720

acgtttttca ccttagcatt ttgtgacttt tgtcgaaagc tgcttttcca gggtttccgc 780

tgtcaaacat gtggttataa atttcaccag cgttgtagta cagaagttcc actgatgtgt 840

gttaattatg accaacttga tttgctgttt gtctccaagt tctttgaaca ccacccaata 900
ccacaggaag aggcgtcctt agcagagact gccctaacat ctggatcatc cccttccgca 960
cccgcctcgg actctattgg gccccaaatt ctcaccagtc cgtctccttc aaaatccatt 1020
ccaattccac agcccttccg accagcagat gaagatcatc gaaatcaatt tgggcaacga 1080
gaccgatcct catcagctcc caatgtgcat ataaacacaa tagaacctgt caatattgat 1140
gacttgatta gagaccaagg atttcgtggt gatggaggat caaccacagg tttgtctgct 1200
accccccctg cctcattacc tggctcacta actaacgtga aagccttaca gaaatctcca 1260
ggacctcagc gagaaaggaa gtcatcttca tcctcagaag acaggaatcg aatgaaaaca 1320
cttggtagac gggactcgag tgatgattgg gagattcctg atgggcagat tacagtggga 1380
caaagaattg gatctggatc atttggaaca gtctacaagg gaaagtggca tggtgatgtg 1440
gcagtgaaaa tgttgaatgt gacagcacct acacctcagc agttacaagc cttcaaaaat 1500
gaagtaggag tactcaggaa aacacgacat gtgaatatcc tactcttcat gggctattcc 1560
acaaagccac aactggctat tgttacccag tggtgtgagg gctccagctt gtatcaccat 1620
ctccatatca ttgagaccaa atttgagatg atcaaactta tagatattgc acgacagact 1680
gcacagggca tggattactt acacgccaag tcaatcatcc acagagacct caagagtaat 1740
aatatatttc ttcatgaaga cctcacagta aaaataggtg attttggtct agctacagtg 1800
aaatctcgat ggagtgggtc ccatcagttt gaacagttgt ctggatccat tttgtggatg 1860
gcaccagaag tcatcagaat gcaagataaa aatccataca gctttcagtc agatgtatat 1920
gcatttggaa ttgttctgta tgaattgatg actggacagt taccttattc aaacatcaac 1980
aacagggacc agataatttt tatggtggga cgaggatacc tgtctccaga tctcagtaag 2040
gtacggagta actgtccaaa agccatgaag agattaatgg cagagtgcct caaaaagaaa 2100
agagatgaga gaccactctt tccccaaatt ctcgcctcta ttgagctgct ggcccgctca 2160
ttgccaaaaa ttcaccgcag tgcatcagaa ccctccttga atcgggctgg tttccaaaca 2220
gaggatttta gtctatatgc ttgtgcttct ccaaaaacac ccatccaggc agggggatat 2280
ggtgcgtttc ctgtccactg aaacaaatga gtgagagagt tcaggagagt agcaacaaaa 2340
ggaaaataaa tgaacatatg tttgcttata tgttaaattg aataaaatac tctctttttt 2400
tttaaggtga accaaagaac acttgtgtgg ttaaagacta gatataattt ttccccaaac 2460
taaaatttat acttaacatt ggatttttaa catccaaggg ttaaaataca tagacattgc 2520
taaaaattgg cagagcctct tctagaggct ttactttctg ttccgggttt gtatcattca 2580
cttggttatt ttaagtagta aacttcagtt tctcatgcaa cttttgttgc cagctatcac 2640

atgtccacta gggactccag aagaagaccc tacctatgcc tgtgtttgca ggtgagaagt 2700

tggcagtcgg ttagcctggg ttagataagg caaactgaac agatctaatt taggaagtca 2760

gtagaattta ataattctat tattattctt aataattttt ctataactat ttctttttat 2820

aacaatttgg aaaatgtgga tgtcttttat ttccttgaag caataaacta agtttctttt 2880

tataaaaa 2888

<210> 4
<211> 3147
<212> DNA
<213> Homo sapiens

<400> 4
   atggctactc aagctgattt gatggagttg gacatggcca tggaaccaga cagaaaagcg 60
   gctgttagtc actggcagca acagtcttac ctggactctg gaatccattc tggtgccact 120
   accacagctc cttctctgag tggtaaaggc aatcctgagg aagaggatgt ggatacctcc 180
   caagtcctgt atgagtggga acagggattt tctcagtcct tcactcaaga acaagtagct 240
   gatattgatg gacagtatgc aatgactcga gctcagaggg tacgagctgc tatgttccct 300
   gagacattag atgagggcat gcagatccca tctacacagt ttgatgctgc tcatcccact 360
   aatgtccagc gtttggctga accatcacag atgctgaaac atgcagttgt aaacttgatt 420
   aactatcaag atgatgcaga acttgccaca cgtgcaatcc ctgaactgac aaaactgcta 480
   aatgacgagg accaggtggt ggttaataag gctgcagtta tggtccatca gctttctaaa 540
   aaggaagctt ccagacacgc tatcatgcgt tctcctcaga tggtgtctgc tattgtacgt 600
   accatgcaga atacaaatga tgtagaaaca gctcgttgta ccgctgggac cttgcataac 660
   ctttcccatc atcgtgaggg cttactggcc atctttaagt ctggaggcat tcctgccctg 720
   gtgaaaatgc ttggttcacc agtggattct gtgttgtttt atgccattac aactctccac 780
   aaccttttat tacatcaaga aggagctaaa atggcagtgc gtttagctgg tgggctgcag 840
   aaaatggttg ccttgctcaa caaaacaaat gttaaattct tggctattac gacagactgc 900
   cttcaaattt tagcttatgg caaccaagaa agcaagctca tcatactggc tagtggtgga 960
   ccccaagctt tagtaaatat aatgaggacc tatacttacg aaaaactact gtggaccaca 1020
   agcagagtgc tgaaggtgct atctgtctgc tctagtaata agccggctat tgtagaagct 1080
   ggtggaatgc aagctttagg acttcacctg acagatccaa gtcaacgtct tgttcagaac 1140
   tgtctttgga ctctcaggaa tctttcagat gctgcaacta aacaggaagg gatggaaggt 1200
   ctccttggga ctcttgttca gcttctgggt tcagatgata taaatgtggt cacctgtgca 1260
   gctggaattc tttctaacct cacttgcaat aattataaga acaagatgat ggtctgccaa 1320
   gtgggtggta tagaggctct tgtgcgtact gtccttcggg ctggtgacag ggaagacatc 1380
   actgagcctg ccatctgtgc tcttcgtcat ctgaccagcc gacaccaaga agcagagatg 1440
   gcccagaatg cagttcgcct tcactatgga ctaccagttg tggttaagct cttacaccca 1500
   ccatcccact ggcctctgat aaaggctact gttggattga ttcgaaatct tgccctttgt 1560
   cccgcaaatc atgcaccttt gcgtgagcag ggtgccattc cacgactagt tcagttgctt 1620
   gttcgtgcac atcaggatac ccagcgccgt acgtccatgg gtgggacaca gcagcaattt 1680
   gtggaggggg tccgcatgga agaaatagtt gaaggttgta ccggagccct tcacatccta 1740
   gctcgggatg ttcacaaccg aattgttatc agaggactaa ataccattcc attgtttgtg 1800
   cagctgcttt attctcccat tgaaaacatc caaagagtag ctgcaggggt cctctgtgaa 1860
   cttgctcagg acaaggaagc tgcagaagct attgaagctg agggagccac agctcctctg 1920
   acagagttac ttcactctag gaatgaaggt gtggcgacat atgcagctgc tgttttgttc 1980
   cgaatgtctg aggacaagcc acaagattac aagaaacggc tttcagttga gctgaccagc 2040
   tctctcttca gaacagagcc aatggcttgg aatgagactg ctgatcttgg acttgatatt 2100
   ggtgcccagg gagaacccct tggatatcgc caggatgatc ctagctatcg ttcttttcac 2160
   tctggtggat atggccagga tgccttgggt atggacccca tgatggaaca tgagatgggt 2220
   ggccaccacc ctggtgctga ctatccagtt gatgggctgc cagatctggg gcatgcccag 2280
   gacctcatgg atgggctgcc tccaggtgac agcaatcagc tggcctggtt tgatactgac 2340
   ctgtaaatca tcctttagct gtattgtctg aacttgcatt gtgattggcc tgtagagttg 2400
   ctgagagggc tcgaggggtg ggctggtatc tcagaaagtg cctgacacac taaccaagct 2460
   gagtttccta tgggaacaat tgaagtaaac tttttgttct ggtccttttt ggtcgaggag 2520
   taacaataca aatggatttt gggagtgact caagaagtga agaatgcaca agaatggatc 2580
   acaagatgga atttatcaaa ccctagcctt gcttgttaaa tttttttttt ttttttttta 2640
   agaatatctg taatggtact gactttgctt gctttgaagt agctcttttt tttttttttt 2700
   tttttttttt gcagtaactg ttttttaagt ctctcgtagt gttaagttat agtgaatact 2760
   gctacagcaa tttctaattt ttaagaattg agtaatggtg tagaacacta attcataatc 2820
   actctaatta attgtaatct gaataaagtg taacaattgt gtagcctttt tgtataaaat 2880
   agacaaatag aaaatggtcc aattagtttc ctttttaata tgcttaaaat aagcaggtgg 2940
   atctatttca tgtttttgat caaaaactat ttgggatatg tatgggtagg gtaaatcagt 3000
   aagaggtgtt atttggaacc ttgttttgga cagtttacca gttgcctttt atcccaaagt 3060
   tgttgtaacc tgctgtgata cgatgcttca agagaaaatg cggttataaa aaatggttca 3120
   gaattaaact tttaattcat tcgattg 3147

<210> 5
<211> 4207
<212> DNA
<213> Homo sapiens

<400> 5
atgactgagt acaaactggt ggtggttgga gcaggtggtg ttgggaaaag cgcactgaca 60

atccagctaa tccagaacca ctttgtagat gaatatgatc ccaccataga ggattcttac 120

agaaaacaag tggttataga tggtgaaacc tgtttgttgg acatactgga tacagctgga 180

caagaagagt acagtgccat gagagaccaa tacatgagga caggcgaagg cttcctctgt 240

gtatttgcca tcaataatag caagtcattt gcggatatta acctctacag ggagcagatt 300

aagcgagtaa aagactcgga tgatgtacct atggtgctag tgggaaacaa gtgtgatttg 360

ccaacaagga cagttgatac aaaacaagcc cacgaactgg ccaagagtta cgggattcca 420

ttcattgaaa cctcagccaa gaccagacag ggtgttgaag atgcttttta cacactggta 480

agagaaatac gccagtaccg aatgaaaaaa ctcaacagca gtgatgatgg gactcagggt 540

tgtatgggat tgccatgtgt ggtgatgtaa caagatactt ttaaagtttt gtcagaaaag 600

agccactttc aagctgcact gacaccctgg tcctgacttc cctggaggag aagtattcct 660

gttgctgtct tcagtctcac agagaagctc ctgctacttc cccagctctc agtagtttag 720

tacaataatc tctatttgag aagttctcag aataactacc tcctcacttg gctgtctgac 780

cagagaatgc acctcttgtt actccctgtt atttttctgc cctgggttct tccacagcac 840

aaacacacct ctgccacccc aggtttttca tctgaaaagc agttcatgtc tgaaacagag 900

aaccaaaccg caaacgtgaa attctattga aaacagtgtc ttgagctcta aagtagcaac 960

tgctggtgat tttttttttc tttttactgt tgaacttaga actatgctaa tttttggaga 1020

aatgtcataa attactgttt tgccaagaat atagttatta ttgctgtttg gtttgtttat 1080

aatgttatcg gctctattct ctaaactggc atctgctcta gattcataaa tacaaaaatg 1140

aatactgaat tttgagtcta tcctagtctt cacaactttg acgtaattaa atccaacttt 1200

cacagtgaag tgcctttttc ctagaagtgg tttgtagact tcctttataa tatttcagtg 1260

gaatagatgt ctcaaaaatc cttatgcatg aaatgaatgt ctgagatacg tctgtgactt 1320

atctaccatt gaaggaaagc tatatctatt tgagagcaga tgccattttg tacatgtatg 1380

aaattggttt tccagaggcc tgttttgggg ctttcccagg agaaagatga aactgaaagc 1440

acatgaataa tttcacttaa taatttttac ctaatctcca cttttttcat aggttactac 1500

ctatacaatg tatgtaattt gtttccccta gcttactgat aaacctaata ttcaatgaac 1560

ttccatttgt attcaaattt gtgtcatacc agaaagctct acatttgcag atgttcaaat 1620

attgtaaaac tttggtgcat tgttatttaa tagctgtgat cagtgatttt caaacctcaa 1680

atatagtata ttaacaaatt acattttcac tgtatatcat ggtatcttaa tgatgtatat 1740

aattgccttc aatccccttc tcaccccacc ctctacagct tcccccacag caataggggc 1800

ttgattattt cagttgagta aagcatggtg ctaatggacc agggtcacag tttcaaaact 1860

tgaacaatcc agttagcatc acagagaaag aaattcttct gcatttgctc attgcaccag 1920

taactccagc tagtaatttt gctaggtagc tgcagttagc cctgcaagga aagaagaggt 1980

cagttagcac aaacccttta ccatgactgg aaaactcagt atcacgtatt taaacatttt 2040

tttttctttt agccatgtag aaactctaaa ttaagccaat attctcattt gagaatgagg 2100

atgtctcagc tgagaaacgt tttaaattct ctttattcat aatgttcttt gaagggttta 2160

aaacaagatg ttgataaatc taagctgatg agtttgctca aaacaggaag ttgaaattgt 2220

tgagacagga atggaaaata taattaattg atacctatga ggatttggag gcttggcatt 2280

ttaatttgca gataataccc tggtaattct catgaaaaat agacttggat aacttttgat 2340

aaaagactaa ttccaaaatg gccactttgt tcctgtcttt aatatctaaa tacttactga 2400

ggtcctccat cttctatatt atgaattttc atttattaag caaatgtcat attaccttga 2460

aattcagaag agaagaaaca tatactgtgt ccagagtata atgaacctgc agagttgtgc 2520

ttcttactgc taattctggg agctttcaca gtactgtcat catttgtaaa tggaaattct 2580

gcttttctgt ttctgctcct tctggagcag tgctactctg taattttcct gaggcttatc 2640

acctcagtca tttctttttt aaatgtctgt gactggcagt gattcttttt cttaaaaatc 2700

tattaaattt gatgtcaaat tagggagaaa gatagttact catcttgggc tcttgtgcca 2760

atagcccttg tatgtatgta cttagagttt tccaagtatg ttctaagcac agaagtttct 2820

aaatggggcc aaaattcaga cttgagtatg ttctttgaat accttaagaa gttacaatta 2880

gccgggcatg gtggcccgtg cctgtagtcc cagctacttg agaggctgag gcaggagaat 2940

cacttcaacc caggaggtgg aggttacagt gagcagagat cgtgccactg cactccagcc 3000

tgggtgacaa gagagacttg tctccaaaaa aaaagttaca cctaggtgtg aattttggca 3060

caaaggagtg acaaacttat agttaaaagc tgaataactt cagtgtggta taaaacgtgg 3120

tttttaggct atgtttgtga ttgctgaaaa gaattctagt ttacctcaaa atccttctct 3180

ttccccaaat taagtgcctg gccagctgtc ataaattaca tattcctttt ggttttttta 3240

aaggttacat gttcaagagt gaaaataaga tgttctgtct gaaggctacc atgccggatc 3300

tgtaaatgaa cctgttaaat gctgtatttg ctccaacggc ttactataga atgttactta 3360

atacaatatc atacttatta caatttttac tataggagtg taataggtaa aattaatctc 3420

tattttagtg ggcccatgtt tagtctttca ccatccttta aactgctgtg aatttttttg 3480

tcatgacttg aaagcaagga tagagaaaca ctttagagat atgtggggtt tttttaccat 3540

tccagagctt gtgagcataa tcatatttgc tttatattta tagtcatgaa ctcctaagtt 3600

ggcagctaca accaagaacc aaaaaatggt gcgttctgct tcttgtaatt catctctgct 3660

aataaattat aagaagcaag gaaaattagg gaaaatattt tatttggatg gtttctataa 3720

acaagggact ataattcttg tacattattt ttcatctttg ctgtttcttt gagcagtcta 3780

atgtgccaca caattatcta aggtatttgt tttctataag aattgtttta aaagtattct 3840

tgttaccaga gtagttgtat tatatttcaa aacgtaagat gatttttaaa agcctgagta 3900

ctgacctaag atggaattgt atgaactctg ctctggaggg aggggaggat gtccgtggaa 3960

gttgtaagac ttttattttt ttgtgccatc aaatataggt aaaaataatt gtgcaattct 4020

gctgtttaaa caggaactat tggcctcctt ggccctaaat ggaagggccg atattttaag 4080

ttgattattt tattgtaaat taatccaacc tagttctttt taatttggtt gaatgttttt 4140

tcttgttaaa tgatgtttaa aaaataaaaa ctggaagttc ttggcttagt cataattctt 4200

atattca 4207

## Description

The invention relates, among other aspects, to a method for predicting of response/nonresponse to the treatment with an anti-EGFR antibody, including cetuximab and panitumumab, in patients with colorectal cancer of all UICC stages.

### Introduction

### Colorectal Cancer and Treatment Options

Colorectal cancer affects 73.000 patients in Germany and approx. 145.000 patients in the United States. It is the second most frequent solid tumor after breast and prostate cancer. Treatment of patients with colorectal cancer differs dependent on the location of the tumor, the stage of the disease, various additional risk factors and routine practice in various countries. Standard treatment for patients with colon cancer that is locally defined (stage I and stage II or has spread only to lymph nodes (stage III) always involves surgery to remove the primary tumor. Standard treatment for patients with rectum cancer may differ from country to country and from hospital to hospital as a significant part of these patients will receive neo-adjuvant radio/chemotherapy followed by surgery to remove the tumor tissue.

The five year survival rates of patients with colorectal cancer depend on the clinical stage of the individual patient, the histopathological diagnosis, stage-specific treatment options as well as on routine medical practice that differs from country to country, and often also from hospital to hospital. There are also significant differences in the routine treatment of patient with colorectal cancer in the Western world.

In most countries, patients with UICC stage disease will not receive any additional chemotherapy after surgery as their five year survival is approximately 95 %.

Treatment options for patients with UICC stage II colon cancer differ in many Western countries. The five year survival of patients with UICC II disease is approx. 80-82 %, meaning that 18-20 % will experience a progression of disease - often liver or lung metastasis. Once the disease will have spread to distant organs the outcome of the patients is much worse, and the majority of these patient will die relatively quickly despite heavy treatment of these patients. Therefore guidelines in some Western countries recommend offering adjuvant chemotherapies to patients with UICC II disease including 5-flourouracil and leucovorine or in combination with oxaliplatin. In other European countries including Germany the guidelines do not recommend to offer patients with UICC II disease adjuvant chemotherapy. There is a controversy if adjuvant chemotherapy should be offered to UICC II patients or not. Randomized clinical data that show a benefit of adjuvant chemotherapy is still missing for these patient cohorts.

Patients with locally advanced colorectal cancer - lymph nodes are infiltrated with cancer cells - have a five year survival rate of 49 %. The treatment guidelines therefore recommend that after surgery all patients should receive adjuvant chemotherapy, either a triple combination of 5-FU, leucovorin and oxaliplatin (FOLFOX4 or FOLFOX6 regimes) or dual combination of capecitabine - an orally available 5-FU derivative - and oxaliplatin (CAPOX). For elderly patients with low ECOG performance scores or know toxicities the dual 5-FU/leucovorin scheme should be used. In the routine practice only 60-80 % of patients with UICC stage III disease will however receive adjuvant chemotherapy. In Germany, only 60 % of UICC III patients will be treated with FOLFOX or 5-FU/leucovorin. There is also a difference in treatment between low densities areas and city populations. In general, approximately 50 % of patients with UICC III disease will experience progression of disease within 1-2 years after surgery. Once distant metatastasis is diagnosed, these patients will be offered additional therapies including treatment with targeted antibodies drugs that inhibit the EGFR receptor including cetuximab or panitumumab or antibodies directed against the VGFA ligand (bevacizimab). Several lines of therapies are offered, but most of these patients with disease progression will die within a five year interval.

The five year survival rate for patients with advanced, metastatic disease is dramatically low. Only 8 % will survive the first five years after surgery. It is these patients for which most of the treatment options with targeted therapies were developed over the last ten years, however, with limited success. The first targeted antibody therapy involved an anti-EGFR antibody cetuximab that was approved in 2004 by the FDA as monotherapy or in combination with Irinotecan, for patients with metastatic CRC (mCRC) that failed prior chemotherapy with irinotecan. In the original BOND study the response rate of the patients for the cetuximab was approximately 11 %. In 2007, a second anti- EGFR antibody, panitumumab, was approved for the treatment of mCRC patients. However, the FDA approved panitumumab only in combination with KRAS wildtype (wt) mutation status, as it was shown in 2007 that only patients with wt KRAS gene would benefit from panitumumab. However, the data also showed that many patients with mCRC and wt KRAS did not benefit from panitumumab. Also, there were some mCRC patients with mutations in the KRAS gene that showed response to panitumumab. Similar data was also published in 2008-2009 for cetuximab that led to a label change for the approval of cetuximab. At the moment, both cetuximab and panitumumab are only approved for patients with mCRC and wildtype KRAS status.

### Companion Diagnostics (CDx)

Accurate prediction of response/nonresponse to therapy is a prerequisite for individualized approaches to treatment. Current clinical practice in the treatment of patients with solid tumors does not offer effective and accurate prediction of response/nonresponse to chemotherapy and hormone therapy.

In prostate cancer no predictive biomarkers are known or established that predict response to radiation, hormone therapy or chemotherapy with taxol. The same is true for advanced non-small cell lung cancer (NSCLC). Approximately 70-80 % of all NSCLC patients are in stage IIIB or stage IV at the time of first diagnosis. For the majority of these patients no predictive markers exist that allow prediction of response to small molecule drugs like erlotinib or iressa that inhibit the kinase function of the EGF receptor. Only in a small cohort of NSCLC patients with EGFR mutations in the kinase domain response to erlotinib was observed. Still 90 % of the NSCLC patients of stage IIIB and IV have a five year survival of less than 8 % despite treatment.

The situation in breast cancer is more complex. For example, most patients with early breast cancer (node negative, ER) will receive radiation, chemotherapy and hormone therapy with tamoxifen after surgical removal of the tumor. The 5 year survival of these patient cohorts is between 90-95 %. However, only 4 % of the patients will benefit from the addition of chemotherapy. Current treatment guidelines still recommend the overtreatment of 100 % of these patients with chemotherapy in order to reach the 4 % patients that may benefit. Similarly, a significant portion of the patients do not benefit from tamoxifen although there are ER positive. Effective methods to predict response to the chemotherapy or hormone therapy are still missing.

There is one FDA approved CDx in breast cancer. Determination of the HERII status is predictive of response to trastuzumab, an anti HERII antibody. Thus patients with HERII positive breast cancer will receive trastuzumab at some point of their treatment history. However, only 25 % of all breast cancer patients are HERII positive and of those only 20-25 % of the patients benefit from trastuzumab, meaning that 75-80 % of HERII positive breast cancer patients are over treated and have no benefit from this expensive treatment.

In colorectal cancer, no predictive biomarkers are established in the adjuvant treatment of UICC II or UICC III patients.

### Clinical Need for CDx in CRC UICC stage II and stage III

At time of first diagnosis, 70 % of the CRC patients are in UICC stage II and UICC stage III. 20 % of the UICC stage II and 49 % of the UICC stage III patients will suffer from progression of disease within 1-2 years after surgery. The majority of the relapsed patients will receive metastasis in the liver followed by metastasis in the lung. Hence, anti-EGFR antibody drugs like cetuximab and panitumumab would be ideal drugs to treat these patients before metastasis will occur if responders to these drugs could be identified and separated from non-responders. Recently, two randomized phase III trials, one in the US and one in Europe, evaluating cetuximab vs. cetuximab plus FOLFOX in UICC stage III patients did not meet their endpoints. Secondary endpoint analysis showed that patients with wild type KRAS did not benefit in the Cetuximab/FOLFOX arm in comparison to patients in the FOLFOX arm (ASCO 2010).

Therefore, there is a large clinical need in the art to predict whether a patient with colorectal cancer will respond to a treatment with an anti-EGFR antibody or not.

### Description of the invention

The inventors discovered combinations of genes that can be used to predict whether a human subject (patient) in need of treatment with an anti-EGFR antibody will respond to such treatment.

Examples for anti-EGFR antibodies that can be used according to the invention are cetuximab, panitumumab, and IMC-11F8. Commercially available Anti-EGFR antibodies are sold and developed, e.g. by Merck-Serono, Bristol-Myer Squibbs (BMS), and Amgen. Anti-EGFR antibodies are developed by other companies, e.g. by Eli Lilly.

Cetuximab (IMC-C225, Erbitux^{®}) is a chimeric (mouse/human) monoclonal antibody, an epidermal growth factor receptor (EGFR) inhibitor, usually given by intravenous infusion. Cetuximab is administered for the treatment of cancer, in particular fro treatment of metastatic colorectal cancer (stage Dukes D) and head and neck cancer.

Cetuximab binds specifically to the extracellular domain of the human epidermal growth factor receptor. It is composed of the Fv regions of a murine anti-EGFR antibody with human IgG1 heavy and kappa light chain constant regions and has an approximate molecular weight of 152 kDa. Cetuximab is produced in mammalian (murine myeloma) cell culture.

Panitumumab, also known as ABX-EGF, is a fully human monoclonal antibody specific to the epidermal growth factor receptor (EGFR). Panitumumab is manufactured by Amgen and sold as Vectibix^{®}.

IMC-11F8 is a potent, fully human monoclonal antibody that targets the epidermal growth factor receptor (EGFR). It is currently in Phase II studies for metastatic colorectal cancer with one or more Phase III trials planned in 2009. IMC-11F8 is in development by Eli Lilly.

Specifically, the inventors found that the absence or presence of certain mutations in the sequence of the genes PIK3CA, KRAS, and BRAF allows for the prediction whether a subject in need of anti-EGFR antibody treatment will respond to such an anti-EGFR antibody treatment or not. Accordingly, the mutation status of these genes is determined. The mutation status can either be wild type (unaltered) or mutated. Any change of nucleotide in a nucleic acid or of an amino acid in a protein is referred to as a mutation.

The sequence of the mRNAs transcribed from the genes PIK3CA, KRAS, and BRAF is described herein as SEQ ID NOs 1, 2, and 3, respectively.

KRAS and its principal effector BRAF are important proteins in the EGFR signaling pathway and are involved in promotion of tumor growth, spread and differentiation. The PIK3CA gene encodes for the p110α protein, the class I PI-3-kinase catalytic subunit.

The mutations in the genes PIK3CA, KRAS, BRAF, CTNNB1, and NRAS that can be determined according to the invention are as follows:

**Table A**

| **Gene** | **Mutation nt** | **Mutation aa** |
|---|---|---|
| PIK3CA | 1624G>A | E542K (Glu -> Lys) |
| PIK3CA | 1633G>A | E545K (Glu -> Lys) |
| PIK3CA | 3140A>G | H1047R (His - Arg) |
| KRAS | 34G>A | G12S (Gly -> Ser) |
| KRAS | 34G>T | G12C (Gly -> Cys) |
| KRAS | 34G>C | G12R (Gly -> Arg) |
| KRAS | 35G>A | G12D (Gly -> Asp) |
| KRAS | 35G>T | G12V (Gly -> Val) |
| KRAS | 35G>C | G12A (Gly -> Ala) |
| KRAS | 37G>T | G13C (Gly -> Cys) |
| KRAS | 38G>A | G13D (Gly -> Asp) |
| KRAS | 436G>A | A146T (Ala -> Thr) |
| KRAS | 181C>A | Q61K (Gln -> Lys) |
| KRAS | 181C>G | Q61E (Gln -> Glu) |
| KRAS | 182A>C | Q61P (Gln -> Pro) |
| KRAS | 182A>G | Q61R (Gln -> Arg) |
| KRAS | 182A>T | Q61L (Gln -> Leu) |
| KRAS | 183A>C | Q61H (Gln -> His) |
| KRAS | 183A>T | Q61H (Gln -> His) |
| BRAF | 1799T>A | V600E (Val -> Glu) |
| CTNNB1 | 121A>G | T41A (Thr -> Ala) |
| CTNNB1 | 134C>T | S45F (Ser -> Phe) |
| NRAS | 34G>A | G12S (Gly -> Ser) |
| NRAS | 34G>T | G12C (Gly -> Cys) |
| NRAS | 35G>A | G12D (Gly -> Asp) |
| NRAS | 35G>t | G12V (Gly -> Val) |
| NRAS | 37G>C | G13R (Gly -> Arg) |
| NRAS | 38G>A | G13D (Gly -> Asp) |
| NRAS | 181C>A | Q61K (Gln -> Lys) |
| NRAS | 182A>G | Q61R (Gln -> Arg) |
| NRAS | 182A>T | Q61L (Gln -> Leu) |

In another aspect of the invention, the mutation status of a gene can also be deduced from the protein sequence of the protein encoded by the particular gene (given in table A). The protein sequence of the proteins of the invention are known in the art an can also be deduced from the nucleic acid sequence provided herein.

In a preferred embodiment, the human patient is clinically "responding" to anti-EGFR antibody treatment as used herein when the T/C value is < 20. The T/C value represents the treated-to-control ratio of relative median tumor volumes.

The invention refers, in a first aspect, to a method for predicting the responsiveness of a human patient in need of anti-EGFR antibody treatment to such treatment based on a biological sample. Such a method comprises the following two steps. Firstly, the mutation status of at least PIK3CA, KRAS, and BRAF is determined in the biological sample, and secondly, it is concluded based on the mutation status of PIK3CA, KRAS, and BRAF, whether the subject is responsive or non-responsive to anti-EGFR antibody treatment. The term responsiveness relates to whether a subject will be respond or not respond to a treatment with anti-EGFR antibody.

When determining the mutation status of PIK3CA, the method comprises determining whether at least one mutation chosen from the group consisting of 1624G>A, 1633G>A, and 3140A>G is present in the biological sample.

When determining the mutation status of KRAS the method comprises determining whether at least one mutations chosen from the group consisting of 34G>A, 34G>T, 34G>C, 35G>A, 35G>T, 35G>C, 37G>T, 38 G>A, 181C>A, 181C>G, 182A>C, 182A>G, 182A>T, 183A>C, 183A>T, and 436 G>A is present in the biological sample.

When determining the mutation status of BRAF the method comprises determining whether a T>A mutation is present at position 1799 of the BRAF gene.

A wild type (wt) mutation status of PIK3CA, KRAS, and BRAF allows concluding that the subject is likely to respond to anti-EGFR antibody treatment.

Specifically, in a preferred embodiment, a subject that is found to have a mutation status = wild type in:
the gene PIK3CA at positions 1624G, 1633G, and 3140A,
the gene KRAS at positions 34G, 35G, 38G and 436G, and
the gene BRAF at position 1799T
is a responder to anti-EGFR antibody treatment with a sensitivity of 83 % and a specificity of 76 %.

In an alternative, preferred embodiment, a subject that is found to have a mutation status = wild type in:
the gene PIK3CA at positions 1624G, 1633G, and 3140A, and
the gene KRAS at positions 34G, 35G, 38G and 436G, and
the gene BRAF at position 1799T, and
a mutation status = 35G>A in the gene KRAS
is a responder to anti-EGFR antibody treatment with a sensitivity of 94 % and a specificity of 67 %.

If a mutation status of one ore more of the mutations
PIK3CA 1624G>A, 1633G>A, 3140A>G
KRAS 34G>A, 34G>T, 34G>C, 35G>T, 35G>C, 38 G>A, 436 G>A
BRAF 1799T>A
is determined based on the tumor sample from the subject in the method, then it is concluded that the subject is a non-responder to anti-EGFR antibody treatment, wherein any single mutation of the ones listed above allows for this conclusion.

In this context, reference is made to tables 3A, 3B and 4.

Colorectal cancer (CRC) refers to both colorectal adenoma and colorectal carcinoma. A colorectal adenoma is characterized by atypical growth of epithelial cells in mucosal tissue, i.e. neoplasia. Hypercellularity with enlarged, hyperchromatic nuclei, varying degrees of nuclear stratification, nuclear polymorphisms, and loss of polarity are the histologically defining features. In colorectal adenoma, this abnormal growth is confined to the mucosa; a synonym of adenoma is intraepithelial neoplasia (IEN). If this atypical growth of epithelial cells extends/invades through the muscularis mucosae, the muscle layer under the mucosa, with destruction of the usual anatomical wall, the pathologist terms this atypical growth a colorectal carcinoma.

The colorectal cancer can be of any of stage according to the "Union International Contre le Cancer" (UICC). This allows for the testing of cetuximab responsiveness even in cancer stages that cetuximab is not approved for (e.g. cetuximab is approved only for the treatment colorectal cancer of UICC-stage IV). It is preferred that the subject is in either UICC stage II, stage III or stage IV.

UICC-stage 0 includes CIS only. UICC-stages I and II are comprised of the localized stages, whereas UICC-stage III describes CRC where tumor cells have metastasized into regional lymph nodes. The worst case is UICC-stage IV; it describes CRC which has metastasized into other organ(s), usually liver (~75 %), peritoneum (~22 %), and/or lung (~21 %).

The colorectal cancer can be of any of Dukes stage A to D. This allows for the testing of anti EGFR antibody responsiveness even in cancer stages that the anti EGFR antibody is not approved for (e.g., cetuximab is approved only for the treatment of colorectal cancer of the stage Dukes D).

Dukes A describes a colorectal tumor that has not penetrated the wall and has no nodal involvement. Dukes B involves a penetrating cancer through the rectal wall but with no nodal involvement. Dukes C indicates lymph node involvement. Dukes D includes metastases to distant organs.

Further, it is preferred that the subject is chemo-naive at the time the tumor tissue sample is obtained for using the method.

Preferably, the determination of the mutation status of the subject's tumor sample is performed using any molecule from which the mutation status of a gene can be deduced, such as genomic DNA, cDNA, mRNA, cRNA, or its fragments.

For the determination of the mutation status, any method known in the art suitable for finding mutations can be used, such as sequencing, real-time polymerase chain reaction (real-time PCR), Scorpions PCR, High Resolution Melt (HRM) analysis, COLD-PCR, Allel Specific PCR, Single-Stranded Conformation Polymorphism Analysis, Denaturating High Performance Liquid Chromatography, ligation chain reaction (LCR), Northern blotting, or microarray technology. Molecules that can be used for real-time PCR can be found in the examples.

In one embodiment of the invention, any two, more preferred any three or more genes of the group consisting of PIK3CA, KRAS, BRAF, CTNNB1, and/or NRAS are at least used to predict the responsiveness of a human cancer patient to anti-EGFR antibody treatment.

In another embodiment of the invention, human cancer patients may involve patients with colorectal cancer of Dukes A stage (UICC I), Dukes B stage (UICC II), Dukes C stage (UICC III), and Dukes D stage (UICC IV).

In another preferred embodiment of the invention human cancer patients may involve patients with colorectal cancer of Dukes B stage (UICC II) and Dukes C stage (UICC III).

In another aspect, the invention refers to a microarray for predicting the responsiveness of a human patient in need of anti-EGFR antibody treatment to such anti-EGFR antibody treatment. Such a microarray comprises or consists of probes for determining the mutation status of PIK3CA, KRAS, and BRAF in a biological sample, optionally also for determining the mutation status of CTNNB1, and/or NRAS.

A "microarray" includes a specific set of probes, such as oligonucleotides and/or cDNAs (*e.g.*, expressed sequence tags, "ESTs") corresponding in whole or in part, and/or continuously or discontinuously, to regions of genomic DNA or RNAs that can be extracted from a biological sample of a human patient. The probes are bound to a solid support. The support may be selected from beads (magnetic, paramagnetic, etc.), glass slides, and silicon wafers. The probes can correspond in sequence to the genes of the invention such that hybridization between the gene from the subject sample (genomic DNA or cDNA derived therefrom, etc.) and the probe occurs. In the microarray embodiments, the sample genomic DNA, mRNA etc. can optionally be amplified before hybridization to the microarray. Prior to hybridization, the sample genomic DNA, mRNA etc. is fluorescently labeled. Upon hybridization to the array and excitation at the appropriate wavelength, fluorescence emission is quantified. Fluorescence emission for each particular genomic DNA, mRNA etc. is directly correlated with the amount of the particular genomic DNA, mRNA etc. in the sample. The signal can be detected and together with its location on the support can be used to determine which probes hybridized with genomic DNA, mRNA etc. from the subject's biological sample.

Accordingly, in certain aspects, the invention is directed to a kit or microarray for detecting the mutation status in the subject's tumor sample, where this "status" allows for the conclusion of whether the subject will be responding or is likely to respond to anti-EGFR antibody treatment. Probes are designed to minimize cross reactivity and false positives and allow under appropriate conditions for the detection of single mutation. Thus, the invention in certain aspects provides a microarray, which generally comprises a solid support and a set of oligonucleotide probes.

In another aspect, the invention relates to a kit for predicting the responsiveness of a human patient in need of anti-EGFR antibody treatment to such anti-EGFR antibody treatment. Such a kit comprises means for determining the mutation status of at least PIK3CA, KRAS, and BRAF in a biological sample.

The means for determining the mutation status of PIK3CA, KRAS, and/or BRAF, as well as optionally CTNNB1, and/or NRAS are chosen from the group consisting of primers, probes, and blocker oligonucleotides (examples given in Tables 5A and 5B). These means are suitable in particular for use in sequencing, real-time polymerase chain reaction, ligation chain reaction, Northern blotting, or microarray technology. The sequence of the mRNAs transcribed from the genes CTNNB1, and NRAS is described herein as SEQ ID NOs 4, and 5, respectively.

In another aspect, the invention relates to the use of a composition for predicting responsiveness of a human patient in need of anti-EGFR antibody treatment to anti-EGFR antibody treatment. Such a composition comprises or consists of a first molecule from which the mutation status of PIK3CA can be deduced, a second molecule from which the mutation status of KRAS can be deduced, and a third molecule from which the mutation status of BRAF can be deduced.

The first molecule, the second molecule, and/or the third molecule are preferably genomic DNA, cDNA, mRNA, or cRNA or its fragments, such that using sequencing, real-time polymerase chain reaction, ligation chain reaction, Northern blotting, or microarray technology and the like, their nucleotide sequence can at least in part be determined. Based on the determined nucleotide sequence, the mutation status, a prediction can be made whether a subject from whom the composition is derived will be responsive to anti-EGFR antibody treatment.

In another aspect, the invention relates to the use of a stable cancer xenograft that was obtained by transplanting a fresh human tumor sample from a patient with colorectal cancer ontoo a mouse, in particular onto a nude mouse, and to propagate the tumor through several passages. Such engrafted mice can then be used in therapy experiments with for instance anti-EGFR antibodies. The tumor tissue from these mice can then be used for identifying genes that are suited for predicting the responsiveness of a human patient in need of anti-EGFR antibody treatment. Other drugs can also be used, such as bevacizumab and/or oxaliplatin. Such mouse cancer xenografts are accepted models in the art for human cancer research.

In summary, a panel of 149 well-characterized patient-derived CRC xenografts was established. The xenograft models highly resemble the original primary tumor with respect to histology and genome-wide gene expression profiling.

The inventors have examined the mutation status in the three genes KRAS, BRAF and PIK3CA as a tool for the prediction of response/nonresponse towards monotherapy with an anti-EGFR antibody, e.g. cetuximab. The inventors' companion diagnostics (CDx) predicted response with sensitivity of 83 % and nonresponse with a specificity of 76 %. If exon 13 mutations are also linked with CE response the CDx has a sensitivity of 94 % and a specificity of 67 %.

KRAS, BRAF, and PIK3CA mutation status combined has clinical utility higher than KRAS alone to individualize cetuximab treatment, also in the adjuvant setting, for patients with colorectal cancer of Dukes B and Dukes C.

### Examples

### MATERIAL AND METHODS

### Experimental Aim

Human tumor xenografts directly derived from patient cancer tissue are of vital importance for preclinical research as they realistically represent the heterogeneity and individuality of each malignoma with revealed similarity to the original patient carcinoma.

The inventors established 149 stably passageable colorectal cancer (CRC) xenograft models directly from fresh surgical colorectal tumor material. The xenografts provide a relevant model system that is close to the clinical situation and allow testing of antitumor agents in a fast and standardized manner. In 75 xenograft models, the inventors tested this approach and investigated the response/nonresponse towards targeted antibodies approved for the treatment of patients with advanced, metastatic CRC including cetuximab and bevacizumab as well as the standard chemotherapy agent oxaliplatin.

Bevacizumab (Avastin^{®}) is a drug that blocks angiogenesis. It is used to treat various cancers, including colorectal cancer. Bevacizumab is a humanized monoclonal antibody that binds to vascular endothelial growth factor A (VEGF-A), which stimulates angiogenesis. Oxaliplatin (Eloxatin^{®}, Oxaliplatin Medac^{®}), is [(1R,2R)-cyclohexane-1,2-diamine](ethanedioato-O,O')platinum(II) and is known in the art as a cancer chemotherapy drug.

Cetuximab (IMC-C225, Erbitux^{®}) is a chimeric (mouse/human) monoclonal antibody, an epidermal growth factor receptor (EGFR) inhibitor.

Xenograft models provide sufficient tissue material for molecular studies of biomarkers that are predictive for response/nonresponse to therapy and can be used as companion diagnostics (CDx). In 67 xenograft models, the inventors analyzed mutation status of KRAS, BRAF and PIK3CA and investigated their relationships with various clinical and pathological characteristics as well as the response to therapies.

### Patients

All fresh human tumor tissue samples originated from a multi-center prospective study "Molecular Signatures in Colorectal Cancer" (MSKK). 239 tumor tissue samples from patients of all four Dukes stages (UICCI, II, III and IV) were collected during two years by a collaborating network of four clinics, using a standardized procedure. In parallel, comprehensive clinical data were collected for each patient and monitored. All included patients gave written informed consent prior to surgery. Clinical characteristics of 67 chemonaive patients with primary CRC are listed in table 2.

### Establishment of xenografts

Shortly after surgery, original colorectal cancer tumor pieces were shipped in gentamicin containing RPMI-1640 medium to the mouse facility. After arrival at the mouse facilities they were transplanted onto immunodeficient mice and were further passaged until a stably grown tumor xenografts has developed. In this way a panel of 149 stably passagable colon cancer xenografts could be established as a permanent tumor models.

Surgical colorectal tumor samples were cut into pieces of 3 to 4 mm and transplanted within 30 min s.c. to 3 to 6 immunodeficient NOD/SCID mice (Taconic); the gender of the mice was chosen according to the donor patient. Additional tissue samples were immediately snap-frozen and stored at -80°C for genetic, genomic, and protein analyses. All animal experiments were done in accordance with the United Kingdom Co-ordinating Committee on Cancer Research regulations for the Welfare of Animals and of the German Animal Protection Law and approved by the local responsible authorities. Mice were observed daily for tumor growth. At a size of about 1 cm3, tumors were removed and passaged to naive NMRI: *nu*/*nu* mice (Charles River) for chemosensitivity testing. Tumors were passaged no more than 10 times. Numerous samples from early passages were stored in the tissue bank in liquid nitrogen and used for further experiments. Several rethawings led to successful engraftment in nude mice. All xenografts as well as the corresponding primary tumors were subjected to histological evaluation using snap-frozen, haematoxylin-eosin-stained tissue sections.

### Testing of colorectal cancer drugs

75 xenograft models were used in therapy experiments testing responsiveness towards drugs approved in the treatment of patients with colorectal cancer including cetuximab as an anti-EGRF antibody, bevacizumab, and oxaliplatin. Each of the 75 tumors was transplanted onto 20 mice (5 controls and 5 for each drug). Models with treated-to-control ratios of relative median tumor volumes of 20 % or lower were defined as responders. This resulted in response rates of 24 % for cetuximab, 4 % for bevacizumab and 7 % for oxaliplatin (Table 1). The chemotherapeutic response of the passagable tumors was determined in male NMRI: *nu*/*nu* mice. For that purpose, one tumor fragment each was transplanted s.c. to a number of mice. At palpable tumor size (50-100 mm³), 6 to 8 mice each were randomized to treatment and control groups and treatment was initiated. If not otherwise mentioned, the following drugs and treatment modalities were used: Bevacizumab (Avastin®; Genentech Inc., South San Francisco, CA, USA) 50 mg/kg/d, qd 7x2, I.p., Cetuximab (Erbitux; Merck) 50 mg/kg/d, qd 7x2, i.p.; Oxaliplatin (Eloxatin, Sanofi-Avensis), 50 mg/kg/d, qd1-5, I.p. Doses and schedules were chosen according to previous experience in animal experiments and represent the maximum tolerated or efficient doses. The injection volume was 0.2 mL/20 g body weight.

Tumor size was measured in two dimensions twice weekly with a caliper-like instrument. Individual tumor volumes (*V*) were calculated by the formula: *V* = (length + [width]2) / 2 and related to the values at the first day of treatment (relative tumor volume). Median treated to control (T/C) values of relative tumor volume were used for the evaluation of each treatment modality and categorized according to scores (- to ++++; see Table 3). The mean tumor doubling time of each xenograft model was calculated by comparing the size between 2- and 4-fold relative tumor volumes. Statistical analyses were done with the *U* test (Mann and Whitney) with *P* < 0.05. The body weight of mice was determined every 3 to 4 days and the change in body weight was taken as variable for tolerability.

### Molecular characterization of Human Tumor Xenograft Samples

### DNA and RNA extraction

Genomic DNA and total RNA were simultaneously extracted with AllPrep DNA/RNA Mini Kit (*automated* protocol *using* the *QIACube*) according to the manufacturer's instructions. DNA and RNA concentrations (ng/µl) were measured using UV spectrophotometer (Nanovue, GE Healthcare).

### Mutation Analysis

Mutation analysis was conducted by allele-specific real-time PCR for KRAS, BRAF, PIK3CA, APC, CTNNβ1 and TP53 mutations with Custom TaqMan SNP Genotyping Assays (Applied Biosystems). TaqManMGB assays were designed for: 8 substitutions in the KRAS gene (34G>A, 34G>T, 34G>C, 35G>A, 35G>T, 35G>C, 38 G>A and 436 G>A), the most frequent mutation in the BRAF gene (1799 T>A), 3 hotspots in the PIK3CA gene (1624G>A, 1633G>A, 3140A>G), most common substitution in the APC gene (4348 C->T), 2 hotspots in CTNNβ1 (121 A>G, 134 C>T) and 5 substitutions in TP53 (524 G>A, 742 C>T, 743 G>A, 818G>A, 844 C>T). Every well contained 20 ng of purified gDNA template. Samples were run in duplicates in the final volume of 10 µl with TaqMan Universal PCR Master Mix (2x) No AmpErase UNG, 40 x working stock of SNP Genotyping Assay and blocking oligonucleotides (125 nmol/l). Competitive blocking oligonucleotides were added due to the presence of unspecific fluorescence signals originating from the adjacent mutations in codon 12 of the KRAS gene. Amplification was run in the ABI *Fast 7500* instrument. Standard TaqMan thermocycling conditions were used (10 min - 95°C, 40 cycles of. 15 s at 92 °C, 1 min at 60 °C) for KRAS mutation analysis, while for the BRAF anneal/extend step was prolonged to 90 s. A subset of critical samples was confirmed via dideoxy sequencing.

Primers, reporters and Blockers used in the real-time PCR are shown in table 5A and 5B.

Mutation status was assessed in tissue of 67 tumor xenografts, derived from chemonaive patients with primary tumors. The analysis was performed by allele-specific RT-PCR (Custom TaqMan SNP Genotyping Assays, Applied Biosystems) for the following hotspots: KRAS: 34G>A, 34G>T, 34G>C, 35G>A, 35G>T, 35G>C, 38 G>A and 436 G>A;
BRAF: 1799 T>A, and
PIK3CA: 1624G>A, 1633G>A, 3140A>G.

Altogether, the inventors observed at least one mutation in 40 of the 67 (60 %) xenograft models. There were 32 single mutations: 24 in KRAS, 6 in BRAF and 2 in PIK3CA. In 8 of the 67 (12 %) CRC models, the inventors observed mutations in two genes, 7 in KRAS and PIK3CA and 1 in BRAF and PIK3CA. KRAS and BRAF mutations were mutually exclusive.

Mutations in codon 12 of KRAS were most frequent and observed in 21 (31 %) of the models. Of these the 35 G>T mutation occurred in 11 models. There were 6 mutations in codon 13 (9 %) and 2 mutations in codon 146 (3 %) of KRAS. No difference was observed in the frequency of PIK3CA mutations between exons 9 (7 %) and 20 (7 %).

The corresponding primary patient tumors showed the same mutation profile.

### Controls

Every 96-well plate was composed of 40 patients samples in duplicates, 4 negative controls (WT), 8 positive controls (titrations), 4 non - template controls (NTC, DNA substituted with DNase-free water).

As negative control (WT) served genomic DNA isolated from lymphocytes of peripheral blood system of a healthy donor that underwent colonoscopy. As positive control we used serial dilutions of DNA isolated from the cell lines harboring mutations in the investigated hotspots (A549 - KRAS 34 G>A; NCI-H385 - KRAS 34 G>T; HuP-T3 - KRAS 34 G>C; A427 - KRAS 35 G>A, CTNNβ1 121 A>G; SW620 - KRAS 35 G>T, TP53 818 G>A; RPMI-8226 - KRAS 35 G>C; HCT-116 - KRAS 38 G>A; ML-2 - KRAS 436 G>A; HT-29 - BRAF 1799 T>A; T84 - PIK3CA 1624 G>A; HCT-15 - PIK3CA 1633 G>A; SKOV3 - PIK3CA 3140 A>G; KLE - TP53 524 G >A; OVCAR3 - TP53 743 G>A, HuP-T3 - TP53 844 C>T) in wild-type genomic DNA (see above) that mimics different tumor cell content in the sample (100 %; 50 %; 20 %; 10 %; 5 %; 2 %; 1 %; 0.5 %)

### Gene expression analysis - identification of the source tumor of a xenograft based on RNA expression

As part of the validation of the xenograft model, we examined whether the original tumor corresponding to the xenograft was identifiable based on RNA expression patterns. It is our hypothesis that even if this identification is complicated by marked differences between native and xenograft samples, the subset of RNA markers that causes these differences can be identified, such that after exclusion of this subset from the comparison, the identification of the source becomes possible.

As a proxy for the source tumor we use a snap frozen sample removed at the same time as the engrafted material. Identification of the source can then be based on similarities between the xenograft and this original tumor sample. To compare a xenograft to its source, we place each RNA microarray measuring more than 54.000 transcripts in a high dimensional Euclidian space to establish a concept of distance. We consider the source of a xenograft identified, when the xenograft's RNA measurement is closer in distance to the measurement of the native sample from the same source than to all other samples in a sufficiently large comparison set.

The inventors used Affymetrix U133 Plus 2.0 GeneChip measurements from 254 samples, consisting of 127 pairs, each composed of a native sample of a colorectal tumor and one of its xenografts. After condensation of the complete set with FARMS/INI, 18018 informative probesets were identified. To identify probesets that are specific to the difference of xenograft vs. native, we calculated the Wilcoxon rank sum statistic between the group of xenografts and original samples for each probeset.

After removing 20 % of RNA probesets that strongly distinguished the xenograft from the primary tumor. 67 % and more of the tumors were properly identified as pairs that were closer to each other than to any other sample among the 254 (=127*2).

### Response to Cetuximab as an anti-EGFR-antibody

The majority of the cetuximab responders are wild type in KRAS, BRAF and PIK3CA. This corresponds to a sensitivity S+ of 8 3% (15/18). Three of the cetuximab responder (17 %) harbor mutations in the KRAS gene. Of those one mutation was found in codon 12 and two in codon 13. The 38G>A mutation in codon 13 was recently linked with improved response to CE (De Roock et al. JAMA 2010, 304:1812). If the exon 13 mutations are linked with CE response in the inventors' dataset an improved S+ of 94 % will result.

### Nonresponse to Cetuximab as an anti-EGFR-antibody

28 of the 49 cetuximab non-responders exhibited KRAS mutations. If only KRAS mutations are considered 57 % of the non-responders can be identified. The specificity (S-) can be significantly improved to 76 % (37/49) if BRAF and PIK3CA mutations are determined in addition to KRAS mutations (see Tab. 4). If exon 13 mutations are linked with response to cetuximab, S- will decrease to 67 %.

### Prospective Prediction of Response to Cetuximab as an anti-EGFR-antibody

The inventors also investigated how well response can be predicted from the mutation data. A machine learning approach was used to estimate unbiased prospective prediction rates for response to cetuximab based on the mutation profile. In each of a large number of bootstrap loops, a new predictor was trained on a new randomly selected subset of the tumors and applied to the remaining tumors. KRAS, BRAF and PIK3CA mutations achieve a S+ of 84 % (95 % CI: 59 % and 96 %) and a S- of 68 % (95 % CI: 54 % and 80 %).

### Tables

**Tab. 1: Treatment scheme and response rates of the 75 pharmacologically characterized xenograft models. Treatment groups consist of 5 animals each. T/C values represent the treated-to-control ratios of relative median tumor volumes.**

| **Drug** | **Treatment scheme** | **Responder T/C < 20% (> 80% tumor growth inhibition)** | |
|---|---|---|---|
| | | **Number** | **(%)** |
| **Oxaliplatin** | qd1-5 50 mg/kg/d | 5/75 | **7** |
| **Cetuximab** | qd 7x2 50 mg/kg/d | 18/75 | **24** |
| **Bevacizumab** | qd 7x2 50 mg/kg/d | 3/75 | **4** |

**Tab. 2: Mutation distribution in a group of 67 xenograft models in respect to the patient characteristics**

| **Characteristic** | **Total** | **BRAF Mut** | **%** | **KRAS Mut** | **%** | **PIK3CA Mut** | **%** | **Double Mut** | **%** |
|---|---|---|---|---|---|---|---|---|---|
| **Total n pts** | **67** | 7 | 10% | 31 | 46% | 10 | 15% | 8 | 12% |

| **Gender** | | | | | | | | | |
|---|---|---|---|---|---|---|---|---|---|
| **Male** | **31** | 2 | 6% | 12 | 39% | 4 | 13% | 3 | 10% |
| **Female** | **36** | 5 | 14% | 19 | 53% | 6 | 17% | 5 | 14% |

| **Age** | | | | | | | | | |
|---|---|---|---|---|---|---|---|---|---|
| **<60 yrs old** | **15** | 0 | - | 7 | 47% | 2 | 13% | 2 | 13% |
| **>60 yrs old** | **52** | 7 | 13% | 24 | 46% | 8 | 15% | 6 | 12% |

| **Location** | | | | | | | | | |
|---|---|---|---|---|---|---|---|---|---|
| **Colon** | **46** | 7 | 15% | 18 | 39% | 9 | 20% | 7 | 15% |
| **Rectum** | **21** | 0 | - | 13 | 62% | 1 | 5% | 1 | 5% |

| **UICC Stage** | | | | | | | | | |
|---|---|---|---|---|---|---|---|---|---|
| **I** | **8** | 1 | 13% | 5 | 63% | 2 | 25% | 2 | 25% |
| **II** | **22** | 2 | 9% | 5 | 23% | 1 | 5% | 0 | 0% |
| **III** | **28** | 3 | 11% | 18 | 64% | 6 | 21% | 5 | 18% |
| **IV** | **9** | 1 | 11% | 3 | 33% | 1 | 11% | 1 | 11% |

**Tab. 3 A: Mutation status of responders to cetuximab as anti-EGFR antibody: T/C values represent the treated-to-control ratios of relative median tumor volumes; *Rating: If T/C: > 50 %: "-", 36-50 %: "+", 21-35 %: "++", 6-20 %: "+++", < 5 %: "++++"**

| **Model-No.** | **Passage** | **Response to** | | | | | | **Mutation Status** | | |
|---|---|---|---|---|---|---|---|---|---|---|
| | | **Oxaliplatin** | | **Cetuximab** | | **Bevacizumab** | | **KRAS** | **BRAF** | **PIK3CA** |
| | | **T/C** | **Rating** | **T/C** | **Rating** | **T/C** | **Rating** | | | |
| 8169 | P2 | 19,7 | +++ | 7,6 | +++ | 39,4 | + | WT | WT | WT |
| 8233 | P3 | 68,4 | - | 8,9 | +++ | 31,1 | ++ | WT | WT | WT |
| 8439 | P2 | 84,3 | - | 19,6 | +++ | 20,6 | +++ | 38 G>A | WT | WT |
| 8583 | P3 | 65,5 | - | 17,2 | +++ | 43,1 | + | WT | WT | WT |
| 8525 | P3 | 40 | + | 12 | +++ | 38,7 | + | WT | WT | WT |
| 8660 | P3 | 19,4 | +++ | 7 | +++ | 19,4 | +++ | WT | WT | WT |
| 8676 | P3 | 86,4 | - | 17,3 | +++ | 34,9 | ++ | 38 G>A | WT | WT |
| 8445 | P3 | 35,5 | ++ | 5,3 | +++ | 47,4 | + | WT | WT | WT |
| 8704 | P3 | 37,7 | + | 8,6 | +++ | 20,3 | ++ | WT | WT | WT |
| 8754 | P3 | 116,7 | - | 5,3 | +++ | 63,2 | - | WT | WT | WT |
| 8710 | P4 | 37,7 | + | 13,1 | +++ | 41,4 | + | WT | WT | WT |
| 8818 | P4 | 61,0 | - | 17,5 | +++ | 43,9 | + | WT | WT | WT |
| 8060 | P3 | 40,9 | + | 4,5 | ++++ | 46,7 | + | WT | WT | WT |
| 8623 | P2 | 69,3 | - | 2,7 | ++++ | 102,7 | - | WT | WT | WT |
| 8521 | P3 | 68,3 | - | 1,7 | ++++ | 41,0 | + | WT | WT | WT |
| 8555 | P3 | 61,8 | - | 3,6 | ++++ | 80 | - | WT | WT | WT |
| 8858 | P4 | 15,9 | +++ | 1,2 | ++++ | 20,3 | ++ | 34 G>A | WT | WT |
| 8815 | P4 | 66,3 | - | 3,2 | ++++ | 21,0 | ++ | WT | WT | WT |

**Tab. 3 B. Mutation status of non-responders to cetuximab as anti-EGFR antibody: T/C values represent the treated-to-control ratios of relative median tumor volumes; *Rating: If T/C: > 50 %: "-", 36-50 %: "+", 21-35 %: "++", 6-20 %: "+++", < 5 %: "++++"**

| **Model-No.** | **Passage** | **Response to** | | | | | | **Mutation Status** | | |
|---|---|---|---|---|---|---|---|---|---|---|
| | | **Oxaliplatin** | | **Cetuximab** | | **Bevacizumab** | | **KRAS** | **BRAF** | **PIK3CA** |
| | | **T/C** | **Rating** | **T/C** | **Rating** | **T/C** | **Rating** | | | |
| 8260 | P2 | 61,5 | - | 72,7 | - | 66,6 | - | WT | 1799 T>A | WT |
| 8213 | P3 | 54,7 | - | 94,7 | - | 63,8 | - | WT | 1799 T>A | WT |
| 8183 | P3 | 72,9 | - | 67,8 | - | 103,4 | - | 35 G>A | WT | WT |
| 8433 | P2 | 34,7 | ++ | 79,2 | - | 23,6 | ++ | 38 G>A | WT | WT |
| 8434 | P2 | 93 | - | 61 | - | 88,2 | - | 35 G>T | WT | 3140 A>G |
| 8435 | P2 | 85,9 | - | 81,4 | - | 37,2 | + | 436 G>A | WT | 1633 G>A |
| 8424 | P2 | 58,3 | - | 58,3 | - | 33,3 | ++ | WT | WT | WT |
| 8469 | P2 | 68,4 | - | 55,2 | - | 57,9 | - | 35 G>T | WT | WT |
| 8610 | P2 | 53,6 | - | 62,5 | - | 50 | + | 35 G>A | WT | WT |
| 8649 | P2 | 71 | - | 79,3 | - | 82,8 | - | 35 G>T | WT | 1633 G>A |
| 8683 | P2 | 90,3 | - | 120 | - | 70,3 | - | 35 G>T | WT | 1633 G>A |
| 8611 | P2 | 71,4 | - | 74,7 | - | 88,7 | - | 35 G>T | WT | WT |
| 8522 | P3 | 37 | + | 64,4 | - | 38 | + | WT | WT | WT |
| 8632 | P3 | 30,2 | ++ | 62,5 | - | 87,5 | - | WT | WT | WT |
| 8734 | P3 | 83,3 | - | 79,2 | - | 67,8 | - | WT | WT | 1624 G>A |
| 8722 | P3 | 90 | - | 73,1 | - | 56 | - | WT | WT | WT |
| 8421 | P3 | 95,3 | - | 81 | - | 54,2 | - | WT | 1799 T>A | WT |
| 8537 | P3 | 37 | + | 60,9 | - | 57,2 | - | WT | 1799 T>A | WT |
| 8764 | P3 | 77,3 | - | 82,1 | - | 54,6 | - | 38 G>A | WT | WT |
| 8729 | P3 | 31,5 | ++ | 53,2 | - | 64,3 | - | 35 G>T | WT | WT |
| 8428 | P3 | 75 | - | 88 | - | 66,7 | - | 35 G>T | WT | WT |
| 8690 | P3 | 52,2 | - | 89,7 | - | 53,6 | - | WT | WT | WT |
| 8732 | P3 | 67,7 | - | 51,6 | - | 47,4 | + | 38 G>A | WT | WT |
| 8823 | P2 | 43,2 | + | 69,3 | - | 31,4 | ++ | WT | 1799 T>A | 3140 A>G |
| 8723 | P3 | 60,9 | - | 65,7 | - | 50 | + | 34 G>A | WT | 3140 A>G |
| 8833 | P3 | 48,5 | + | 69,7 | - | 54,6 | - | WT | 1799 T>A | WT |
| 8795 | P3 | 61,5 | - | 51,2 | - | 58,2 - | | 35 G>T | WT | 3140 A>G |
| 8814 | P3 | 38,8 | + | 63,4 | - | 38,4 | + | WT | 1799 T>A | WT |
| 8847 | P3 | 88,3 | - | 93 | - | 34,5 | ++ | 35 G>T | WT | WT |
| 8207 | P2 | 56,7 | - | 43,7 | + | 24,5 | ++ | WT | WT | WT |
| 8209 | P3 | 42,2 | + | 36,9 | + | 16,5 | +++ | 35 G>A | WT | WT |
| 8440 | P2 | 45,7 | + | 48,6 | + | 41,2 | + | WT | WT | WT |
| 8397 | P2 | 25,4 | ++ | 40,8 | + | 36,6 | + | 35 G>A | WT | WT |
| 8541 | P2 | 76,6 | - | 42,4 | + | 90 | - | WT | WT | WT |
| 8613 | P2 | 52,2 | - | 45,6 | + | 108,7 | - | WT | WT | WT |
| 8509 | P3 | 56,6 | - | 39,6 | + | 50,9 | - | 35 G>C | WT | WT |
| 8497 | P3 | 43,9 | + | 38,1 | + | 40,4 | + | WT | WT | WT |
| 8652 | P3 | 45,2 | + | 46,7 | + | 31 | ++ | WT | WT | WT |
| 8862 | P3 | 22,3 | ++ | 49,2 | + | 21,4 | ++ | 436 G>A | WT | 3140 A>G |
| 8873 | P4 | 28,6 | ++ | 47,7 | + | 52,4 | - | 35 G>T | WT | WT |
| 8724 | P4 | 57,1 | - | 42,7 | + | 22,2 | ++ | 38 G>A | WT | WT |
| 8389 | P3 | 51,5 | + | 24,8 | ++ | 64,6 | - | 35 G>C | WT | WT |
| 8311 | P2 | 22,2 | ++ | 22 | ++ | 26 | ++ | 35 G>C | WT | WT |
| 8578 | P2 | 58,1 | - | 32 | ++ | 26,7 | ++ | 35 G>C | WT | WT |
| 8591 | P2 | 186,2 | - | 35,3 | ++ | 53 | - | WT | WT | WT |
| 8590 | P3 | 62,2 | - | 27 | ++ | 54,5 - | | 35 G>A | WT | WT |
| 8733 | P3 | 4,7 | ++++ | 28 | ++ | 24,32 | ++ | 35 G>T | WT | WT |
| 8784 | P3 | 22,5 | ++ | 27,1 | ++ | 35,42 | ++ | 35 G>C | WT | WT |
| 8760 | P3 | 72,2 | - | 33,8 | ++ | 48,65 | + | WT | WT | 1633 G>A |

**Table 4: Cross tabulation table. It shows the relation between mutation status in 3 analyzed genes (KRAS, BRAF and PIK3CA) and response towards cetuximab as an anti-EGRF antibody. It allows calculating the prediction of CE response with a sensitivity (S+) of 83 % and a specificity (S-) of 76 % (S+: the percentage of responders who are wild type; S-: the percentage of mutated nonresponders). PPV (positive predictive value) = 55 %, NPV (negative predictive value) = 92,5 %**

| **Mutation status** | **Cetuximab response observed** | | **Total** |
|---|---|---|---|
| | **Responders** | **Nonresponders** | |
| **Mutated** | 3 | 37 | 40 |
| **Wildtype** | 15 | 12 | 27 |
| **Total** | 18 | 49 | 67 |

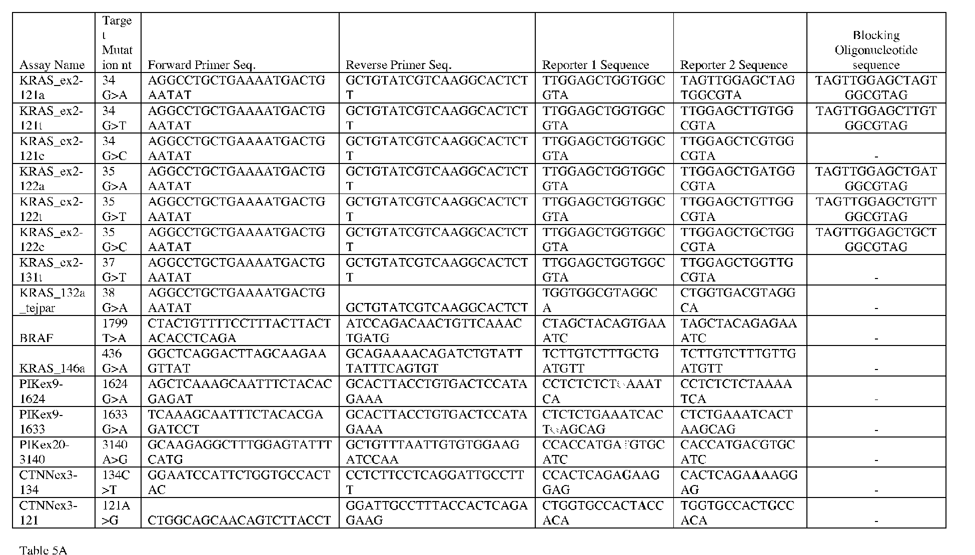

**Table 5B**

| Target Mutation nt | Blocking Oligonucleotide sequence |
|---|---|
| 34 G>A | TAGTTGGAGCTAGTGGCGTAG |
| 34 G>T | TAGTTGGAGCTTGTGGCGTAG |
| 34 G>C | - |
| 35 G>A | TAGTTGGAGCTGATGGCGTAG |
| 35 G>T | TAGTTGGAGCTGTTGGCGTAG |
| 35 G>C | TAGTTGGAGCTGCTGGCGTAG |
| 37 G>T | - |
| 38 G>A | - |
| 1799 T>A | - |
| 436 G>A | - |
| 134C>T | - |
| 121A>G | - |

### Figures

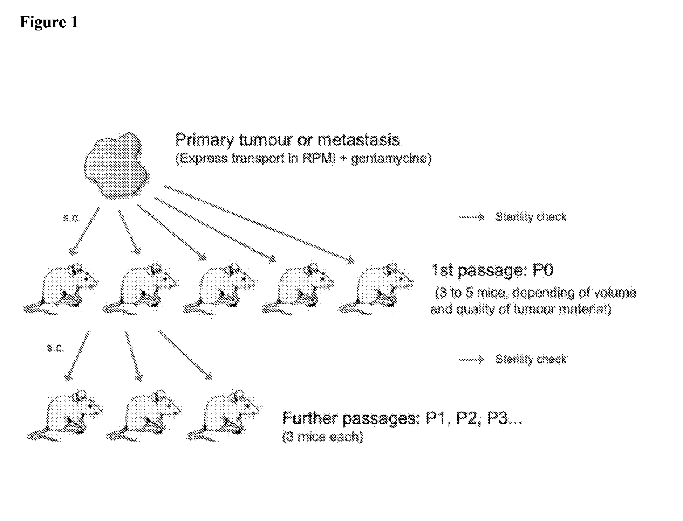

Figure 1: Transplantation of tumor pieces onto highly immunodeficient NOD/SCID mice immediately upon arrival. Passages performed until development of stable growing human xenograft.

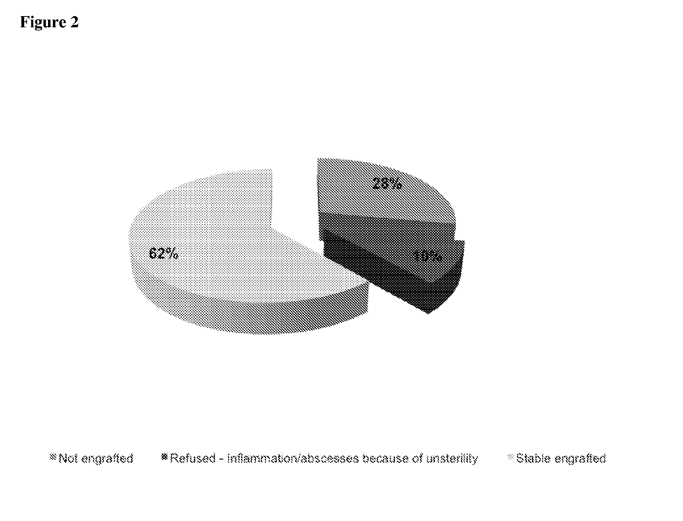

Figure 2: Results of the transplantation of 239 clinical samples. Relatively high engraftment rate of 62 % was obtained. 10 % of samples were refused as a result of unsterility, which is a natural problem of colon tissue.

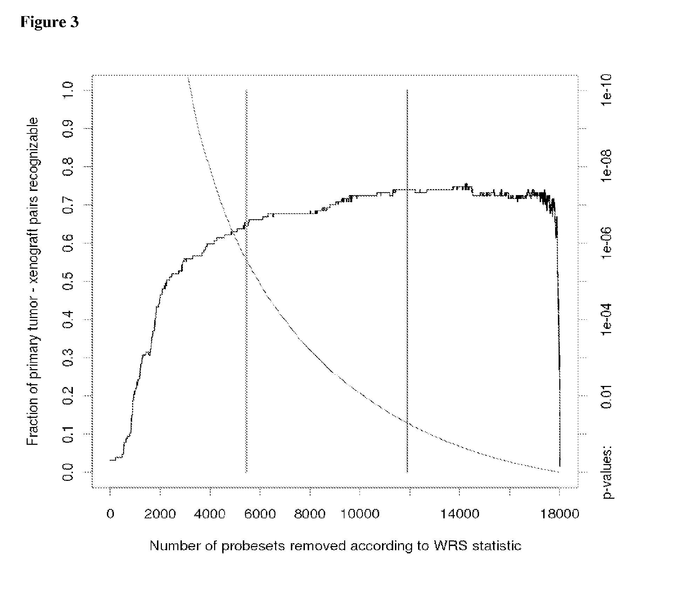

Figure 3: Recognition of primary tumor / xenograft pairs originating from the same patient. The inventors ordered mRNAs by increasing p-value in a Wilcoxon rank sum test for difference between xenografts and primary tumors along the x axis. The falling (lighter) curve shows the p-value (right scale). The right vertical bar marks the p value of 5 %, the left vertical bar marks the Bonferroni adjusted p value for multiple testing. The black curve (scale on the left) shows the fraction of same-source primary tumor/xenograft pairs that were identified after leaving out probesets with p-values below the threshold on the x-axis. Specifically, the x-axis lists all 18018 probesets by increasing p-value in the Wilcox test: it starts on the left with p-values smaller than 1e-10, for the probesets most different between xenografts and native samples. The falling (lighter) curve with its negative-logarithmic scale on the right y-axis shows only the higher p-values as they increase from 1e-10 to 1 - p-values below 1e-10 are omitted. The right vertical bar marks a threshold of 5 % without adjusting for multiple testing of 18018 probesets. A Bonferroni-adjusted threshold is shown as a left vertical bar. The black curve with its scale on the left y-axis, shows the fraction of same-source xenograft-native pairs that were identified after leaving out the number of probesets named on the x-axis. In other word, the x-axis shows the number of probesets that are removed from consideration before source identification is attempted, their removal is due to high native vs. xenograft difference, independent of their value for source characterization.

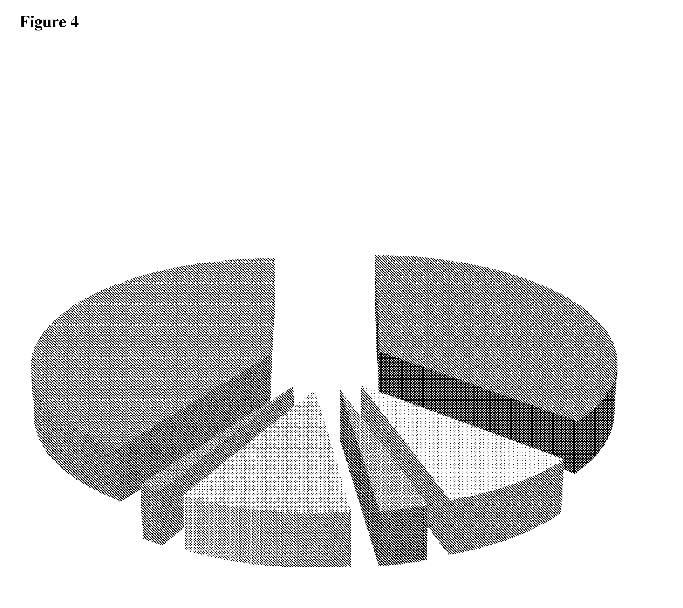

Figure 4: Mutations distribution in 67 xenograft models
   Starting with the top right pie piece, going clockwise:
   24/67 KRAS, 36 %; 6/67 BRAF, 9 %; 2/67 PIK3CA, 3 %; 8/67 PIK3CA + KRAS, 10 %; 1/67 PIK3CA + BRAF, 2 %; 27/67 WT, 40 %.

### Sequences

In the following sequences, nucleotides whose mutation status can be determined according to the invention are written in bold; nucleotides, whose mutation status is preferably being determined according to the invention are written in bold and are also underlined.

Homo sapiens phosphoinositide-3-kinase, catalytic, alpha polypeptide (PIK3CA), mRNA (SEQ ID NO. 1)
ATGCCTCCACGACCATCATCAGGTGAACTGTGGGGCATCCACTTGATGCCCCCAAGAATCCTAGTAGAATGTTTA
CTACCAAATGGAATGATAGTGACTTTAGAATGCCTCCGTGAGGCTACATTAATAACCATAAAGCATGAACTATTT
AAAGAAGCAAGAAAATACCCCCTCCATCAACTTCTTCAAGATGAATCTTCTTACATTTTCGTAAGTGTTACTCAA
GAAGCAGAAAGGGAAGAATTTTTTGATGAAACAAGACGACTTTGTGACCTTCGGCTTTTTCAACCCTTTTTAAAA
GTAATTGAACCAGTAGGCAACCGTGAAGAAAAGATCCTCAATCGAGAAATTGGTTTTGCTATCGGCATGCCAGTG
TGTGAATTTGATATGGTTAAAGATCCAGAAGTACAGGACTTCCGAAGAAATATTCTGAACGTTTGTAAAGAAGCT
GTGGATCTTAGGGACCTCAATTCACCTCATAGTAGAGCAATGTATGTCTATCCTCCAAATGTAGAATCTTCACCA
GAATTGCCAAAGCACATATATAATAAATTAGATAAAGGGCAAATAATAGTGGTGATCTGGGTAATAGTTTCTCCA
AATAATGACAAGCAGAAGTATACTCTGAAAATCAACCATGACTGTGTACCAGAACAAGTAATTGCTGAAGCAATC
AGGAAAAAAACTCGAAGTATGTTGCTATCCTCTGAACAACTAAAACTCTGTGTTTTAGAATATCAGGGCAAGTAT
ATTTTAAAAGTGTGTGGATGTGATGAATACTTCCTAGAAAAATATCCTCTGAGTCAGTATAAGTATATAAGAAGC
TGTATAATGCTTGGGAGGATGCCCAATTTGATGTTGATGGCTAAAGAAAGCCTTTATTCTCAACTGCCAATGGAC
TGTTTTACAATGCCATCTTATTCCAGACGCATTTCCACAGCTACACCATATATGAATGGAGAAACATCTACAAAA
TCCCTTTGGGTTATAAATAGTGCACTCAGAATAAAAATTCTTTGTGCAACCTACGTGAATGTAAATATTCGAGAC
ATTGATAAGATCTATGTTCGAACAGGTATCTACCATGGAGGAGAACCCTTATGTGACAATGTGAACACTCAAAGA
GTACCTTGTTCCAATCCCAGGTGGAATGAATGGCTGAATTATGATATATACATTCCTGATCTTCCTCGTGCTGCT
CGACTTTGCCTTTCCATTTGCTCTGTTAAAGGCCGAAAGGGTGCTAAAGAGGAACACTGTCCATTGGCATGGGGA
AATATAAACTTGTTTGATTACACAGACACTCTAGTATCTGGAAAAATGGCTTTGAATCTTTGGCCAGTACCTCAT
GGATTAGAAGATTTGCTGAACCCTATTGGTGTTACTGGATCAAATCCAAATAAAGAAACTCCATGCTTAGAGTTG
GAGTTTGACTGGTTCAGCAGTGTGGTAAAGTTCCCAGATATGTCAGTGATTGAAGAGCATGCCAATTGGTCTGTA
TCCCGAGAAGCAGGATTTAGCTATTCCCACGCAGGACTGAGTAACAGACTAGCTAGAGACAATGAATTAAGGGAA
AATGACAAAGAACAGCTCAAAGCAATTTCTACACGAGATCCTCTCTCTGAAATCACTGAGCAGGAGAAAGATTTT
CTATGGAGTCACAGACACTATTGTGTAACTATCCCCGAAATTCTACCCAAATTGCTTCTGTCTGTTAAATGGAAT
TCTAGAGATGAAGTAGCCCAGATGTATTGCTTGGTAAAAGATTGGCCTCCAATCAAACCTGAACAGGCTATGGAA
CTTCTGGACTGTAATTACCCAGATCCTATGGTTCGAGGTTTTGCTGTTCGGTGCTTGGAAAAATATTTAACAGAT
GACAAACTTTCTCAGTATTTAATTCAGCTAGTACAGGTCCTAAAATATGAACAATATTTGGATAACTTGCTTGTG
AGATTTTTACTGAAGAAAGCATTGACTAATCAAAGGATTGGGCACTTTTTCTTTTGGCATTTAAAATCTGAGATG
CACAATAAAACAGTTAGCCAGAGGTTTGGCCTGCTTTTGGAGTCCTATTGTCGTGCATGTGGGATGTATTTGAAG
CACCTGAATAGGCAAGTCGAGGCAATGGAAAAGCTCATTAACTTAACTGACATTCTCAAACAGGAGAAGAAGGAT
GAAACACAAAAGGTACAGATGAAGTTTTTAGTTGAGCAAATGAGGCGACCAGATTTCATGGATGCTCTACAGGGC
TTTCTGTCTCCTCTAAACCCTGCTCATCAACTAGGAAACCTCAGGCTTGAAGAGTGTCGAATTATGTCCTCTGCA
AAAAGGCCACTGTGGTTGAATTGGGAGAACCCAGACATCATGTCAGAGTTACTGTTTCAGAACAATGAGATCATC
TTTAAAAATGGGGATGATTTACGGCAAGATATGCTAACACTTCAAATTATTCGTATTATGGAAAATATCTGGCAA
AATCAAGGTCTTGATCTTCGAATGTTACCTTATGGTTGTCTGTCAATCGGTGACTGTGTGGGACTTATTGAGGTG
GTGCGAAATTCTCACACTATTATGCAAATTCAGTGCAAAGGCGGCTTGAAAGGTGCACTGCAGTTCAACAGCCAC
ACACTACATCAGTGGCTCAAAGACAAGAACAAAGGAGAAATATATGATGCAGCCATTGACCTGTTTACACGTTCA
TGTGCTGGATACTGTGTAGCTACCTTCATTTTGGGAATTGGAGATCGTCACAATAGTAACATCATGGTGAAAGAC
GATGGACAACTGTTTCATATAGATTTTGGACACTTTTTGGATCACAAGAAGAAAAAATTTGGTTATAAACGAGAA
CGTGTGCCATTTGTTTTGACACAGGATTTCTTAATAGTGATTAGTAAAGGAGCCCAAGAATGCACAAAGACAAGA
GAATTTGAGAGGTTTCAGGAGATGTGTTACAAGGCTTATCTAGCTATTCGACAGCATGCCAATCTCTTCATAAAT
CTTTTCTCAATGATGCTTGGCTCTGGAATGCCAGAACTACAATCTTTTGATGACATTGCATACATTCGAAAGACC
CTAGCCTTAGATAAAACTGAGCAAGAGGCTTTGGAGTATTTCATGAAACAAATGAATGATGCACATCATGGTGGC
TGGACAACAAAAATGGATTGGATCTTCCACACAATTAAACAGCATGCATTGAACTGAAAAGATAACTGAGAAAAT
GAAAGCTCACTCTGGATTCCACACTGCACTGTTAATAACTCTCAGCAGGCAAAGACCGATTGCATAGGAATTGCA
CAATCCATGAACAGCATTAGAATTTACAGCAAGAACAGAAATAAAATACTATATAATTTAAATAATGTAAACGCA
AACAGGGTTTGATAGCACTTAAACTAGTTCATTTCAAAATTAAGCTTTAGAATAATGCGCAATTTCATGTTATGC
CTTAAGTCCAAAAAGGTAAACTTTGAAGATTGTTTGTATCTTTTTTTAAAAAACAAAACAAAACAAAAATCCCCA
AAATATATAGAAATGATGGAGAAGGAAAAAAAAAAAAAAAAA

Homo sapiens v-Ki-ras2 Kirsten rat sarcoma viral oncogene homolog (KRAS), transcript variant b, mRNA (SEQ ID NO. 2)
ATGACTGAATATAAACTTGTGGTAGTTGGAGCTGGTGGCGTAGGCAAGAGTGCCTTGACGATACAGCTAATTCAG
AATCATTTTGTGGACGAATATGATCCAACAATAGAGGATTCCTACAGGAAGCAAGTAGTAATTGATGGAGAAACC
TGTCTCTTGGATATTCTCGACACAGCAGGTCAAGAGGAGTACAGTGCAATGAGGGACCAGTACATGAGGACTGGG
GAGGGCTTTCTTTGTGTATTTGCCATAAATAATACTAAATCATTTGAAGATATTCACCATTATAGAGAACAAATT
AAAAGAGTTAAGGACTCTGAAGATGTACCTATGGTCCTAGTAGGAAATAAATGTGATTTGCCTTCTAGAACAGTA
GACACAAAACAGGCTCAGGACTTAGCAAGAAGTTATGGAATTCCTTTTATTGAAACATCAGCAAAGACAAGACAG
GGTGTTGATGATGCCTTCTATACATTAGTTCGAGAAATTCGAAAACATAAAGAAAAGATGAGCAAAGATGGTAAA
AAGAAGAAAAAGAAGTCAAAGACAAAGTGTGTAATTATGTAAATACAATTTGTACTTTTTTCTTAAGGCATACTA
GTACAAGTGGTAATTTTTGTACATTACACTAAATTATTAGCATTTGTTTTAGCATTACCTAATTTTTTTCCTGCT
CCATGCAGACTGTTAGCTTTTACCTTAAATGCTTATTTTAAAATGACAGTGGAAGTTTTTTTTTCCTCTAAGTGC
CAGTATTCCCAGAGTTTTGGTTTTTGAACTAGCAATGCCTGTGAAAAAGAAACTGAATACCTAAGATTTCTGTCT
TGGGGTTTTTGGTGCATGCAGTTGATTACTTCTTATTTTTCTTACCAATTGTGAATGTTGGTGTGAAACAAATTA
ATGAAGCTTTTGAATCATCCCTATTCTGTGTTTTATCTAGTCACATAAATGGATTAATTACTAATTTCAGTTGAG
ACCTTCTAATTGGTTTTTACTGAAACATTGAGGGAACACAAATTTATGGGCTTCCTGATGATGATTCTTCTAGGC
ATCATGTCCTATAGTTTGTCATCCCTGATGAATGTAAAGTTACACTGTTCACAAAGGTTTTGTCTCCTTTCCACT
GCTATTAGTCATGGTCACTCTCCCCAAAATATTATATTTTTTCTATAAAAAGAAAAAAATGGAAAAAAATTACAA
GGCAATGGAAACTATTATAAGGCCATTTCCTTTTCACATTAGATAAATTACTATAAAGACTCCTAATAGCTTTTC
CTGTTAAGGCAGACCCAGTATGAAATGGGGATTATTATAGCAACCATTTTGGGGCTATATTTACATGCTACTAAA
TTTTTATAATAATTGAAAAGATTTTAACAAGTATAAAAAATTCTCATAGGAATTAAATGTAGTCTCCCTGTGTCA
GACTGCTCTTTCATAGTATAACTTTAAATCTTTTCTTCAACTTGAGTCTTTGAAGATAGTTTTAATTCTGCTTGT
GACATTAAAAGATTATTTGGGCCAGTTATAGCTTATTAGGTGTTGAAGAGACCAAGGTTGCAAGGCCAGGCCCTG
TGTGAACCTTTGAGCTTTCATAGAGAGTTTCACAGCATGGACTGTGTCCCCACGGTCATCCAGTGTTGTCATGCA
TTGGTTAGTCAAAATGGGGAGGGACTAGGGCAGTTTGGATAGCTCAACAAGATACAATCTCACTCTGTGGTGGTC
CTGCTGACAAATCAAGAGCATTGCTTTTGTTTCTTAAGAAAACAAACTCTTTTTTAAAAATTACTTTTAAATATT
AACTCAAAAGTTGAGATTTTGGGGTGGTGGTGTGCCAAGACATTAATTTTTTTTTTAAACAATGAAGTGAAAAAG
TTTTACAATCTCTAGGTTTGGCTAGTTCTCTTAACACTGGTTAAATTAACATTGCATAAACACTTTTCAAGTCTG
ATCCATATTTAATAATGCTTTAAAATAAAAATAAAAACAATCCTTTTGATAAATTTAAAATGTTACTTATTTTAA
AATAAATGAAGTGAGATGGCATGGTGAGGTGAAAGTATCACTGGACTAGGAAGAAGGTGACTTAGGTTCTAGATA
GGTGTCTTTTAGGACTCTGATTTTGAGGACATCACTTACTATCCATTTCTTCATGTTAAAAGAAGTCATCTCAAA
CTCTTAGTTTTTTTTTTTTACAACTATGTAATTTATATTCCATTTACATAAGGATACACTTATTTGTCAAGCTCA
GCACAATCTGTAAATTTTTAACCTATGTTACACCATCTTCAGTGCCAGTCTTGGGCAAAATTGTGCAAGAGGTGA
AGTTTATATTTGAATATCCATTCTCGTTTTAGGACTCTTCTTCCATATTAGTGTCATCTTGCCTCCCTACCTTCC
ACATGCCCCATGACTTGATGCAGTTTTAATACTTGTAATTCCCCTAACCATAAGATTTACTGCTGCTGTGGATAT
CTCCATGAAGTTTTCCCACTGAGTCACATCAGAAATGCCCTACATCTTATTTCCTCAGGGCTCAAGAGAATCTGA
CAGATACCATAAAGGGATTTGACCTAATCACTAATTTTCAGGTGGTGGCTGATGCTTTGAACATCTCTTTGCTGC
CCAATCCATTAGCGACAGTAGGATTTTTCAAACCTGGTATGAATAGACAGAACCCTATCCAGTGGAAGGAGAATT
TAATAAAGATAGTGCTGAAAGAATTCCTTAGGTAATCTATAACTAGGACTACTCCTGGTAACAGTAATACATTCC
ATTGTTTTAGTAACCAGAAATCTTCATGCAATGAAAAATACTTTAATTCATGAAGCTTACTTTTTTTTTTTGGTG
TCAGAGTCTCGCTCTTGTCACCCAGGCTGGAATGCAGTGGCGCCATCTCAGCTCACTGCAACCTCCATCTCCCAG
GTTCAAGCGATTCTCGTGCCTCGGCCTCCTGAGTAGCTGGGATTACAGGCGTGTGCCACTACACTCAACTAATTT
TTGTATTTTTAGGAGAGACGGGGTTTCACCCTGTTGGCCAGGCTGGTCTCGAACTCCTGACCTCAAGTGATTCAC
CCACCTTGGCCTCATAAACCTGTTTTGCAGAACTCATTTATTCAGCAAATATTTATTGAGTGCCTACCAGATGCC
AGTCACCGCACAAGGCACTGGGTATATGGTATCCCCAAACAAGAGACATAATCCCGGTCCTTAGGTAGTGCTAGT
GTGGTCTGTAATATCTTACTAAGGCCTTTGGTATACGACCCAGAGATAACACGATGCGTATTTTAGTTTTGCAAA
GAAGGGGTTTGGTCTCTGTGCCAGCTCTATAATTGTTTTGCTACGATTCCACTGAAACTCTTCGATCAAGCTACT
TTATGTAAATCACTTCATTGTTTTAAAGGAATAAACTTGATTATATTGTTTTTTTATTTGGCATAACTGTGATTC
TTTTAGGACAATTACTGTACACATTAAGGTGTATGTCAGATATTCATATTGACCCAAATGTGTAATATTCCAGTT
TTCTCTGCATAAGTAATTAAAATATACTTAAAAATTAATAGTTTTATCTGGGTACAAATAAACAGGTGCCTGAAC
TAGTTCACAGACAAGGAAACTTCTATGTAAAAATCACTATGATTTCTGAATTGCTATGTGAAACTACAGATCTTT
GGAACACTGTTTAGGTAGGGTGTTAAGACTTACACAGTACCTCGTTTCTACACAGAGAAAGAAATGGCCATACTT
CAGGAACTGCAGTGCTTATGAGGGGATATTTAGGCCTCTTGAATTTTTGATGTAGATGGGCATTTTTTTAAGGTA
GTGGTTAATTACCTTTATGTGAACTTTGAATGGTTTAACAAAAGATTTGTTTTTGTAGAGATTTTAAAGGGGGAG
AATTCTAGAAATAAATGTTACCTAATTATTACAGCCTTAAAGACAAAAATCCTTGTTGAAGTTTTTTTAAAAAAA
GCTAAATTACATAGACTTAGGCATTAACATGTTTGTGGAAGAATATAGCAGACGTATATTGTATCATTTGAGTGA
ATGTTCCCAAGTAGGCATTCTAGGCTCTATTTAACTGAGTCACACTGCATAGGAATTTAGAACCTAACTTTTATA
GGTTATCAAAACTGTTGTCACCATTGCACAATTTTGTCCTAATATATACATAGAAACTTTGTGGGGCATGTTAAG
TTACAGTTTGCACAAGTTCATCTCATTTGTATTCCATTGATTTTTTTTTTCTTCTAAACATTTTTTCTTCAAACA
GTATATAACTTTTTTTAGGGGATTTTTTTTTAGACAGCAAAAACTATCTGAAGATTTCCATTTGTCAAAAAGTAA

TGATTTCTTGATAATTGTGTAGTAATGTTTTTTAGAACCCAGCAGTTACCTTAAAGCTGAATTTATATTTAGTAA
CTTCTGTGTTAATACTGGATAGCATGAATTCTGCATTGAGAAACTGAATAGCTGTCATAAAATGAAACTTTCTTT
CTAAAGAAAGATACTCACATGAGTTCTTGAAGAATAGTCATAACTAGATTAAGATCTGTGTTTTAGTTTAATAGT
TTGAAGTGCCTGTTTGGGATAATGATAGGTAATTTAGATGAATTTAGGGGAAAAAAAAGTTATCTGCAGATATGT
TGAGGGCCCATCTCTCCCCCCACACCCCCACAGAGCTAACTGGGTTACAGTGTTTTATCCGAAAGTTTCCAATTC
CACTGTCTTGTGTTTTCATGTTGAAAATACTTTTGCATTTTTCCTTTGAGTGCCAATTTCTTACTAGTACTATTT
CTTAATGTAACATGTTTACCTGGAATGTATTTTAACTATTTTTGTATAGTGTAAACTGAAACATGCACATTTTGT
ACATTGTGCTTTCTTTTGTGGGACATATGCAGTGTGATCCAGTTGTTTTCCATCATTTGGTTGCGCTGACCTAGG
AATGTTGGTCATATCAAACATTAAAAATGACCACTCTTTTAATTGAAATTAACTTTTAAATGTTTATAGGAGTAT
GTGCTGTGAAGTGATCTAAAATTTGTAATATTTTTGTCATGAACTGTACTACTCCTAATTATTGTAATGTAATAA
AAATAGTTACAGTGACAAAAAAAAAAAAAAAA

Homo sapiens v-raf murine sarcoma viral oncogene homolog B1 (BRAF), mRNA
(SEQ ID NO. 3)
ATGGCGGCGCTGAGCGGTGGCGGTGGTGGCGGCGCGGAGCCGGGCCAGGCTCTGTTCAACGGGGACATGGAGCCC
GAGGCCGGCGCCGGCGCCGGCGCCGCGGCCTCTTCGGCTGCGGACCCTGCCATTCCGGAGGAGGTGTGGAATATC
AAACAAATGATTAAGTTGACACAGGAACATATAGAGGCCCTATTGGACAAATTTGGTGGGGAGCATAATCCACCA
TCAATATATCTGGAGGCCTATGAAGAATACACCAGCAAGCTAGATGCACTCCAACAAAGAGAACAACAGTTATTG
GAATCTCTGGGGAACGGAACTGATTTTTCTGTTTCTAGCTCTGCATCAATGGATACCGTTACATCTTCTTCCTCT
TCTAGCCTTTCAGTGCTACCTTCATCTCTTTCAGTTTTTCAAAATCCCACAGATGTGGCACGGAGCAACCCCAAG
TCACCACAAAAACCTATCGTTAGAGTCTTCCTGCCCAACAAACAGAGGACAGTGGTACCTGCAAGGTGTGGAGTT
ACAGTCCGAGACAGTCTAAAGAAAGCACTGATGATGAGAGGTCTAATCCCAGAGTGCTGTGCTGTTTACAGAATT
CAGGATGGAGAGAAGAAACCAATTGGTTGGGACACTGATATTTCCTGGCTTACTGGAGAAGAATTGCATGTGGAA
GTGTTGGAGAATGTTCCACTTACAACACACAACTTTGTACGAAAAACGTTTTTCACCTTAGCATTTTGTGACTTT
TGTCGAAAGCTGCTTTTCCAGGGTTTCCGCTGTCAAACATGTGGTTATAAATTTCACCAGCGTTGTAGTACAGAA
GTTCCACTGATGTGTGTTAATTATGACCAACTTGATTTGCTGTTTGTCTCCAAGTTCTTTGAACACCACCCAATA
CCACAGGAAGAGGCGTCCTTAGCAGAGACTGCCCTAACATCTGGATCATCCCCTTCCGCACCCGCCTCGGACTCT
ATTGGGCCCCAAATTCTCACCAGTCCGTCTCCTTCAAAATCCATTCCAATTCCACAGCCCTTCCGACCAGCAGAT
GAAGATCATCGAAATCAATTTGGGCAACGAGACCGATCCTCATCAGCTCCCAATGTGCATATAAACACAATAGAA
CCTGTCAATATTGATGACTTGATTAGAGACCAAGGATTTCGTGGTGATGGAGGATCAACCACAGGTTTGTCTGCT
ACCCCCCCTGCCTCATTACCTGGCTCACTAACTAACGTGAAAGCCTTACAGAAATCTCCAGGACCTCAGCGAGAA
AGGAAGTCATCTTCATCCTCAGAAGACAGGAATCGAATGAAAACACTTGGTAGACGGGACTCGAGTGATGATTGG
GAGATTCCTGATGGGCAGATTACAGTGGGACAAAGAATTGGATCTGGATCATTTGGAACAGTCTACAAGGGAAAG
TGGCATGGTGATGTGGCAGTGAAAATGTTGAATGTGACAGCACCTACACCTCAGCAGTTACAAGCCTTCAAAAAT
GAAGTAGGAGTACTCAGGAAAACACGACATGTGAATATCCTACTCTTCATGGGCTATTCCACAAAGCCACAACTG
GCTATTGTTACCCAGTGGTGTGAGGGCTCCAGCTTGTATCACCATCTCCATATCATTGAGACCAAATTTGAGATG
ATCAAACTTATAGATATTGCACGACAGACTGCACAGGGCATGGATTACTTACACGCCAAGTCAATCATCCACAGA
GACCTCAAGAGTAATAATATATTTCTTCATGAAGACCTCACAGTAAAAATAGGTGATTTTGGTCTAGCTACAGTG
AAATCTCGATGGAGTGGGTCCCATCAGTTTGAACAGTTGTCTGGATCCATTTTGTGGATGGCACCAGAAGTCATC
AGAATGCAAGATAAAAATCCATACAGCTTTCAGTCAGATGTATATGCATTTGGAATTGTTCTGTATGAATTGATG
ACTGGACAGTTACCTTATTCAAACATCAACAACAGGGACCAGATAATTTTTATGGTGGGACGAGGATACCTGTCT
CCAGATCTCAGTAAGGTACGGAGTAACTGTCCAAAAGCCATGAAGAGATTAATGGCAGAGTGCCTCAAAAAGAAA
AGAGATGAGAGACCACTCTTTCCCCAAATTCTCGCCTCTATTGAGCTGCTGGCCCGCTCATTGCCAAAAATTCAC
CGCAGTGCATCAGAACCCTCCTTGAATCGGGCTGGTTTCCAAACAGAGGATTTTAGTCTATATGCTTGTGCTTCT
CCAAAAACACCCATCCAGGCAGGGGGATATGGTGCGTTTCCTGTCCACTGAAACAAATGAGTGAGAGAGTTCAGG
AGAGTAGCAACAAAAGGAAAATAAATGAACATATGTTTGCTTATATGTTAAATTGAATAAAATACTCTCTTTTTT
TTTAAGGTGAACCAAAGAACACTTGTGTGGTTAAAGACTAGATATAATTTTTCCCCAAACTAAAATTTATACTTA
ACATTGGATTTTTAACATCCAAGGGTTAAAATACATAGACATTGCTAAAAATTGGCAGAGCCTCTTCTAGAGGCT
TTACTTTCTGTTCCGGGTTTGTATCATTCACTTGGTTATTTTAAGTAGTAAACTTCAGTTTCTCATGCAACTTTT
GTTGCCAGCTATCACATGTCCACTAGGGACTCCAGAAGAAGACCCTACCTATGCCTGTGTTTGCAGGTGAGAAGT
TGGCAGTCGGTTAGCCTGGGTTAGATAAGGCAAACTGAACAGATCTAATTTAGGAAGTCAGTAGAATTTAATAAT
TCTATTATTATTCTTAATAATTTTTCTATAACTATTTCTTTTTATAACAATTTGGAAAATGTGGATGTCTTTTAT
TTCCTTGAAGCAATAAACTAAGTTTCTTTTTATAAAAA

Homo sapiens catenin (cadherin-associated protein), beta 1, 88kDa (CTNNB1),
transcript variant 2, mRNA (SEQ ID NO. 4)
ATGGCTACTCAAGCTGATTTGATGGAGTTGGACATGGCCATGGAACCAGACAGAAAAGCGGCTGTTAGTCACTGG
CAGCAACAGTCTTACCTGGACTCTGGAATCCATTCTGGTGCCACTACCACAGCTCCTTCTCTGAGTGGTAAAGGC
AATCCTGAGGAAGAGGATGTGGATACCTCCCAAGTCCTGTATGAGTGGGAACAGGGATTTTCTCAGTCCTTCACT
CAAGAACAAGTAGCTGATATTGATGGACAGTATGCAATGACTCGAGCTCAGAGGGTACGAGCTGCTATGTTCCCT
GAGACATTAGATGAGGGCATGCAGATCCCATCTACACAGTTTGATGCTGCTCATCCCACTAATGTCCAGCGTTTG
GCTGAACCATCACAGATGCTGAAACATGCAGTTGTAAACTTGATTAACTATCAAGATGATGCAGAACTTGCCACA
CGTGCAATCCCTGAACTGACAAAACTGCTAAATGACGAGGACCAGGTGGTGGTTAATAAGGCTGCAGTTATGGTC
CATCAGCTTTCTAAAAAGGAAGCTTCCAGACACGCTATCATGCGTTCTCCTCAGATGGTGTCTGCTATTGTACGT
ACCATGCAGAATACAAATGATGTAGAAACAGCTCGTTGTACCGCTGGGACCTTGCATAACCTTTCCCATCATCGT
GAGGGCTTACTGGCCATCTTTAAGTCTGGAGGCATTCCTGCCCTGGTGAAAATGCTTGGTTCACCAGTGGATTCT
GTGTTGTTTTATGCCATTACAACTCTCCACAACCTTTTATTACATCAAGAAGGAGCTAAAATGGCAGTGCGTTTA
GCTGGTGGGCTGCAGAAAATGGTTGCCTTGCTCAACAAAACAAATGTTAAATTCTTGGCTATTACGACAGACTGC
CTTCAAATTTTAGCTTATGGCAACCAAGAAAGCAAGCTCATCATACTGGCTAGTGGTGGACCCCAAGCTTTAGTA
AATATAATGAGGACCTATACTTACGAAAAACTACTGTGGACCACAAGCAGAGTGCTGAAGGTGCTATCTGTCTGC
TCTAGTAATAAGCCGGCTATTGTAGAAGCTGGTGGAATGCAAGCTTTAGGACTTCACCTGACAGATCCAAGTCAA
CGTCTTGTTCAGAACTGTCTTTGGACTCTCAGGAATCTTTCAGATGCTGCAACTAAACAGGAAGGGATGGAAGGT
CTCCTTGGGACTCTTGTTCAGCTTCTGGGTTCAGATGATATAAATGTGGTCACCTGTGCAGCTGGAATTCTTTCT
AACCTCACTTGCAATAATTATAAGAACAAGATGATGGTCTGCCAAGTGGGTGGTATAGAGGCTCTTGTGCGTACT
GTCCTTCGGGCTGGTGACAGGGAAGACATCACTGAGCCTGCCATCTGTGCTCTTCGTCATCTGACCAGCCGACAC
CAAGAAGCAGAGATGGCCCAGAATGCAGTTCGCCTTCACTATGGACTACCAGTTGTGGTTAAGCTCTTACACCCA
CCATCCCACTGGCCTCTGATAAAGGCTACTGTTGGATTGATTCGAAATCTTGCCCTTTGTCCCGCAAATCATGCA
CCTTTGCGTGAGCAGGGTGCCATTCCACGACTAGTTCAGTTGCTTGTTCGTGCACATCAGGATACCCAGCGCCGT
ACGTCCATGGGTGGGACACAGCAGCAATTTGTGGAGGGGGTCCGCATGGAAGAAATAGTTGAAGGTTGTACCGGA
GCCCTTCACATCCTAGCTCGGGATGTTCACAACCGAATTGTTATCAGAGGACTAAATACCATTCCATTGTTTGTG
CAGCTGCTTTATTCTCCCATTGAAAACATCCAAAGAGTAGCTGCAGGGGTCCTCTGTGAACTTGCTCAGGACAAG
GAAGCTGCAGAAGCTATTGAAGCTGAGGGAGCCACAGCTCCTCTGACAGAGTTACTTCACTCTAGGAATGAAGGT
GTGGCGACATATGCAGCTGCTGTTTTGTTCCGAATGTCTGAGGACAAGCCACAAGATTACAAGAAACGGCTTTCA
GTTGAGCTGACCAGCTCTCTCTTCAGAACAGAGCCAATGGCTTGGAATGAGACTGCTGATCTTGGACTTGATATT
GGTGCCCAGGGAGAACCCCTTGGATATCGCCAGGATGATCCTAGCTATCGTTCTTTTCACTCTGGTGGATATGGC
CAGGATGCCTTGGGTATGGACCCCATGATGGAACATGAGATGGGTGGCCACCACCCTGGTGCTGACTATCCAGTT
GATGGGCTGCCAGATCTGGGGCATGCCCAGGACCTCATGGATGGGCTGCCTCCAGGTGACAGCAATCAGCTGGCC
TGGTTTGATACTGACCTGTAAATCATCCTTTAGCTGTATTGTCTGAACTTGCATTGTGATTGGCCTGTAGAGTTG
CTGAGAGGGCTCGAGGGGTGGGCTGGTATCTCAGAAAGTGCCTGACACACTAACCAAGCTGAGTTTCCTATGGGA
ACAATTGAAGTAAACTTTTTGTTCTGGTCCTTTTTGGTCGAGGAGTAACAATACAAATGGATTTTGGGAGTGACT
CAAGAAGTGAAGAATGCACAAGAATGGATCACAAGATGGAATTTATCAAACCCTAGCCTTGCTTGTTAAATTTT
TTTTTTTTTTTTTTAAGAATATCTGTAATGGTACTGACTTTGCTTGCTTTGAAGTAGCTCTTTTTTTTTTTTTTT
TTTTTTTTTGCAGTAACTGTTTTTTAAGTCTCTCGTAGTGTTAAGTTATAGTGAATACTGCTACAGCAATTTCT
AATTTTTAAGAATTGAGTAATGGTGTAGAACACTAATTCATAATCACTCTAATTAATTGTAATCTGAATAAAGTG
TAACAATTGTGTAGCCTTTTTGTATAAAATAGACAAATAGAAAATGGTCCAATTAGTTTCCTTTTTAATATGCTT
AAAATAAGCAGGTGGATCTATTTCATGTTTTTGATCAAAAACTATTTGGGATATGTATGGGTAGGGTAAATCAGT
AAGAGGTGTTATTTGGAACCTTGTTTTGGACAGTTTACCAGTTGCCTTTTATCCCAAAGTTGTTGTAACCTGCTG
TGATACGATGCTTCAAGAGAAAATGCGGTTATAAAAAATGGTTCAGAATTAAACTTTTAATTCATTCGATTG

Homo sapiens neuroblastoma RAS viral (v-ras) oncogene homolog (NRAS), mRNA
(SEQ ID NO. 5)
ATGACTGAGTACAAACTGGTGGTGGTTGGAGCAGGTGGTGTTGGGAAAAGCGCACTGACAATCCAGCTAATCCAG
AACCACTTTGTAGATGAATATGATCCCACCATAGAGGATTCTTACAGAAAACAAGTGGTTATAGATGGTGAAACC
TGTTTGTTGGACATACTGGATACAGCTGGACAAGAAGAGTACAGTGCCATGAGAGACCAATACATGAGGACAGGC
GAAGGCTTCCTCTGTGTATTTGCCATCAATAATAGCAAGTCATTTGCGGATATTAACCTCTACAGGGAGCAGATT
AAGCGAGTAAAAGACTCGGATGATGTACCTATGGTGCTAGTGGGAAACAAGTGTGATTTGCCAACAAGGACAGTT
GATACAAAACAAGCCCACGAACTGGCCAAGAGTTACGGGATTCCATTCATTGAAACCTCAGCCAAGACCAGACAG
GGTGTTGAAGATGCTTTTTACACACTGGTAAGAGAAATACGCCAGTACCGAATGAAAAAACTCAACAGCAGTGAT
GATGGGACTCAGGGTTGTATGGGATTGCCATGTGTGGTGATGTAACAAGATACTTTTAAAGTTTTGTCAGAAAAG
AGCCACTTTCAAGCTGCACTGACACCCTGGTCCTGACTTCCCTGGAGGAGAAGTATTCCTGTTGCTGTCTTCAGT
CTCACAGAGAAGCTCCTGCTACTTCCCCAGCTCTCAGTAGTTTAGTACAATAATCTCTATTTGAGAAGTTCTCAG
AATAACTACCTCCTCACTTGGCTGTCTGACCAGAGAATGCACCTCTTGTTACTCCCTGTTATTTTTCTGCCCTGG
GTTCTTCCACAGCACAAACACACCTCTGCCACCCCAGGTTTTTCATCTGAAAAGCAGTTCATGTCTGAAACAGAG
AACCAAACCGCAAACGTGAAATTCTATTGAAAACAGTGTCTTGAGCTCTAAAGTAGCAACTGCTGGTGATTTTT

TTTTCTTTTTACTGTTGAACTTAGAACTATGCTAATTTTGGAGAAATGTCATAAATTACTGTTTTGCCAAGAAT
ATAGTTATTATTGCTGTTTGGTTTGTTTATAATGTTATCGGCTCTATTCTCTAAACTGGCATCTGCTCTAGATTC
ATAAATACAAAAATGAATACTGAATTTTGAGTCTATCCTAGTCTTCACAACTTTGACGTAATTAAATCCAACTTT
CACAGTGAAGTGCCTTTTTCCTAGAAGTGGTTTGTAGACTTCCTTTATAATATTTCAGTGGAATAGATGTCTCAA
AAATCCTTATGCATGAAATGAATGTCTGAGATACGTCTGTGACTTATCTACCATTGAAGGAAAGCTATATCTATT
TGAGAGCAGATGCCATTTTGTACATGTATGAAATTGGTTTTCCAGAGGCCTGTTTTGGGGCTTTCCCAGGAGAAA
GATGAAACTGAAAGCACATGAATAATTTCACTTAATAATTTTTACCTAATCTCCACTTTTTTCATAGGTTACTAC
CTATACAATGTATGTAATTTGTTTCCCCTAGCTTACTGATAAACCTAATATTCAATGAACTTCCATTTGTATTCA
AATTTGTGTCATACCAGAAAGCTCTACATTTGCAGATGTTCAAATATTGTAAAACTTTGGTGCATTGTTATTTAA
TAGCTGTGATCAGTGATTTTCAAACCTCAAATATAGTATATTAACAAATTACATTTTCACTGTATATCATGGTAT
CTTAATGATGTATATAATTGCCTTCAATCCCCTTCTCACCCCACCCTCTACAGCTTCCCCCACAGCAATAGGGGC
TTGATTATTTCAGTTGAGTAAAGCATGGTGCTAATGGACCAGGGTCACAGTTTCAAAACTTGAACAATCCAGTTA
GCATCACAGAGAAAGAAATTCTTCTGCATTTGCTCATTGCACCAGTAACTCCAGCTAGTAATTTTGCTAGGTAGC
TGCAGTTAGCCCTGCAAGGAAAGAAGAGGTCAGTTAGCACAAACCCTTTACCATGACTGGAAAACTCAGTATCAC
GTATTTAAACATTTTTTTTCTTTTAGCCATGTAGAAACTCTAAATTAAGCCAATATTCTCATTTGAGAATGAGG
ATGTCTCAGCTGAGAAACGTTTTAAATTCTCTTTATTCATAATGTTCTTTGAAGGGTTTAAAACAAGATGTTGAT
AAATCTAAGCTGATGAGTTTGCTCAAAACAGGAAGTTGAAATTGTTGAGACAGGAATGGAAAATATAATTAATTG
ATACCTATGAGGATTTGGAGGCTTGGCATTTTAATTTGCAGATAATACCCTGGTAATTCTCATGAAAAATAGACT
TGGATAACTTTTGATAAAAGACTAATTCCAAAATGGCCACTTTGTTCCTGTCTTTAATATCTAAATACTTACTGA
GGTCCTCCATCTTCTATATTATGAATTTTCATTTATTAAGCAAATGTCATATTACCTTGAAATTCAGAAGAGAAG
AAACATATACTGTGTCCAGAGTATAATGAACCTGCAGAGTTGTGCTTCTTACTGCTAATTCTGGGAGCTTTCACA
GTACTGTCATCATTTGTAAATGGAAATTCTGCTTTTCTGTTTCTGCTCCTTCTGGAGCAGTGCTACTCTGTAATT
TTCCTGAGGCTTATCACCTCAGTCATTTCTTTTTTAAATGTCTGTGACTGGCAGTGATTCTTTTTCTTAAAAATC
TATTAAATTTGATGTCAAATTAGGGAGAAAGATAGTTACTCATCTTGGGCTCTTGTGCCAATAGCCCTTGTATGT
ATGTACTTAGAGTTTTCCAAGTATGTTCTAAGCACAGAAGTTTCTAAATGGGGCCAAAATTCAGACTTGAGTATG
TTCTTTGAATACCTTAAGAAGTTACAATTAGCCGGGCATGGTGGCCCGTGCCTGTAGTCCCAGCTACTTGAGAGG
CTGAGGCAGGAGAATCACTTCAACCCAGGAGGTGGAGGTTACAGTGAGCAGAGATCGTGCCACTGCACTCCAGCC
TGGGTGACAAGAGAGACTTGTCTCCAAAAAAAAAGTTACACCTAGGTGTGAATTTTGGCACAAAGGAGTGACAAA
CTTATAGTTAAAAGCTGAATAACTTCAGTGTGGTATAAAACGTGGTTTTAGGCTATGTTTGTGATTGCTGAAAA
GAATTCTAGTTTACCTCAAAATCCTTCTCTTTCCCCAAATTAAGTGCCTGGCCAGCTGTCATAAATTACATATTC
CTTTTGGTTTTTTTAAAGGTTACATGTTCAAGAGTGAAAATAAGATGTTCTGTCTGAAGGCTACCATGCCGGATC
TGTAAATGAACCTGTTAAATGCTGTATTTGCTCCAACGGCTTACTATAGAATGTTACTTAATACAATATCATACT
TATTACAATTTTTACTATAGGAGTGTAATAGGTAAAATTAATCTCTATTTTAGTGGGCCCATGTTTAGTCTTTCA
CCATCCTTTAAACTGCTGTGAATTTTTTTGTCATGACTTGAAAGCAAGGATAGAGAAACACTTTAGAGATATGTG
GGGTTTTTTTACCATTCCAGAGCTTGTGAGCATAATCATATTTGCTTTATATTTATAGTCATGAACTCCTAAGTT
GGCAGCTACAACCAAGAACCAAAAAATGGTGCGTTCTGCTTCTTGTAATTCATCTCTGCTAATAAATTATAAGAA
GCAAGGAAAATTAGGGAAAATATTTTATTTGGATGGTTTCTATAAACAAGGACTATAATTCTTGTACATTATTT
TTCATCTTTGCTGTTTCTTTGAGCAGTCTAATGTGCCACACAATTATCTAAGGTATTTGTTTTCTATAAGAATTG
TTTTAAAAGTATTCTTGTTACCAGAGTAGTTGTATTATATTTCAAAACGTAAGATGATTTTTAAAAGCCTGAGTA
CTGACCTAAGATGGAATTGTATGAACTCTGCTCTGGAGGGAGGGGAGGATGTCCGTGGAAGTTGTAAGACTTTTA
TTTTTTTGTGCCATCAAATATAGGTAAAAATAATTGTGCAATTCTGCTGTTTAAACAGGAACTATTGGCCTCCTT
GGCCCTAAATGGAAGGGCCGATATTTTAAGTTGATTATTTTATTGTAAATTAATCCAACCTAGTTCTTTTTAATT
TGGTTGAATGTTTTTTCTTGTTAAATGATGTTTAAAAAATAAAAACTGGAAGTTCTTGGCTTAGTCATAATTCTT
ATATTCA

## Claims

1. A method for predicting the responsiveness of a human patient in need of anti-EGFR antibody treatment to such treatment based on a tumor sample, comprising:
- determining the mutation status of PIK3CA, KRAS, and BRAF in the tumor sample, and
- concluding, based on the mutation status of PIK3CA, KRAS, and BRAF, whether the patient is responsive to anti-EGFR antibody treatment.

2. The method of claim 1, wherein determining the mutation status of PIK3CA comprises determining whether at least one mutations is present that is chosen from the group consisting of: 1624G>A, 1633G>A, and 3140A>G.

3. The method of claim 1 or 2, wherein determining the mutation status of KRAS comprises determining whether at least one mutations is present that is chosen from the group consisting of: 34G>A, 34G>T, 34G>C, 35G>A, 35G>T, 35G>C, 38 G>A and 436 G>A.

4. The method of claims 1 to 3, wherein determining the mutation status of BRAF comprises determining whether a T>A mutations is present at position 1799 of the BRAF gene.

5. The method of claims 1 to 4, wherein a wild type mutation status of PIK3CA, KRAS, and BRAF allows concluding that the patient is responding to anti-EGFR antibody treatment.

6. The method of claims 1 to 5, wherein a wild type mutation status of PIK3CA, KRAS, BRAF and KRAS 35G>A mutation allow concluding that the patient is responding to anti-EGFR antibody treatment.

7. The method of claims 1 to 5, wherein at least one mutation in one of the genes PIK3CA, KRAS and BRAF allow concluding that the patient is not responding to anti-EGFR antibody treatment.

8. The method of claims 1 to 7, wherein the anti-EGFR antibody is cetuximab, panitumumab or IMC-11F8.

9. The method of claim 1 to 8, wherein the tumor sample is from a colorectal cancer of any of Dukes stage A to D.

10. The method of claims 1 to 9, wherein determining the mutation status is performed using genomic DNA, cDNA, mRNA, or cRNA or its fragments.

11. The method of claims 1 to 10, wherein determining the mutation status is performed using sequencing, real-time polymerase chain reaction, ligation chain reaction, Northern blotting, High Resolution Melt (HRM) analysis, Scorpions PCR, COLD-PCR, Allele Specific PCR, Single-Stranded Conformation Polymorphism Analysis, Denaturating High Performance Liquid Chromatography or microarray technology.

12. A microarray for predicting the responsiveness of a human patient in need of anti-EGFR antibody treatment to such treatment, comprising:
- probes for determining the mutation status of PIK3CA, KRAS, and BRAF in a biological sample.

13. A kit for predicting the responsiveness of a human patient in need of anti-EGFR antibody treatment to such treatment, comprising:
- means for determining the mutation status of PIK3CA, KRAS, and BRAF in a biological sample.

14. The kit of claim 13, wherein the means for determining the mutation status of PIK3CA, KRAS, and BRAF is chosen from the group consisting of primers, probes, and blocker oligonucleotides, in particular for use in real-time polymerase chain reaction, sequencing, ligation chain reaction, or microarray technology.

15. Use of a composition for predicting responsiveness of a human patient in need of anti-EGFR antibody treatment to such treatment, wherein the composition comprises
- a first molecule from which the mutation status of PIK3CA can be deduced,
- a second molecule from which the mutation status of KRAS can be deduced, and
- a third molecule from which the mutation status of BRAF can be deduced.

16. Use of claim 15, wherein the first molecule, the second molecule, and/or the third molecule are chosen from the group consisting of genomic DNA, cDNA, mRNA, or cRNA or its fragments.

17. Use of a cancer xenograft transplanted onto a mouse for finding genes that can be used for predicting responsiveness of a human patient in need of anti-EGFR antibody treatment to such treatment.
